(19) Europäisches Patentamt
European Patent Office
Office européen des brevets

(11) EP 3 301 194 B1

(12) EUROPEAN PATENT SPECIFICATION

(45) Date of publication and mention of the grant of the patent:
**04.11.2020 Bulletin 2020/45**

(51) Int Cl.:
***C12Q 1/6886*** *(2018.01)*

(21) Application number: **17193918.4**

(22) Date of filing: **29.09.2017**

# (54) METHOD FOR ASSISTING DIAGNOSIS OF ONSET RISK OF GASTRIC CANCER, AND ARTIFICIAL DNA AND KIT FOR DIAGNOSING ONSET RISK OF GASTRIC CANCER USED IN THE METHOD

VERFAHREN ZUR UNTERSTÜTZUNG DER DIAGNOSE DES RISIKOS ZUR ENTSTEHUNG VON MAGENKREBS UND IN DEM VERFAHREN VERWENDETE KÜNSTLICHE DNA UND KIT ZUR DIAGNOSE DES RISIKOS ZUR ENTSTEHUNG VON MAGENKREBS

PROCÉDÉ D'ASSISTANCE AU DIAGNOSTIC DU RISQUE D'APPARITION DU CANCER GASTRIQUE ET ADN ARTIFICIEL ET KIT PERMETTANT DE DIAGNOSTIQUER LE RISQUE D'APPARITION DU CANCER GASTRIQUE UTILISÉS DANS LE PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **30.09.2016 JP 2016192971**
**20.07.2017 JP 2017140546**

(43) Date of publication of application:
**04.04.2018 Bulletin 2018/14**

(73) Proprietors:
- **National Cancer Center**
  **Tokyo 104-0045 (JP)**
- **Sysmex Corporation**
  **Kobe-shi, Hyogo 651-0073 (JP)**

(72) Inventors:
- **Ushijima, Toshikazu**
  **Tokyo 104-0045 (JP)**
- **Maeda, Masahro**
  **Tokyo 104-0045 (JP)**

(74) Representative: **De Clercq & Partners**
**Edgard Gevaertdreef 10a**
**9830 Sint-Martens-Latem (BE)**

(56) References cited:
**WO-A1-2007/145325**

- **JORGE L SEPULVEDA ET AL: "High-definition CpG methylation of novel genes in gastric carcinogenesis identified by next-generation sequencing", MODERN PATHOLOGY, vol. 29, no. 2, 15 January 2016 (2016-01-15), pages 182-193, XP055445614, GB ISSN: 0893-3952, DOI: 10.1038/modpathol.2015.144**
- **HE Q ET AL: "Development of a multiplex MethyLight assay for the detection of multigene methylation in human colorectal cancer", CANCER GENETICS AND CYTOGENETICS, ELSEVIER SCIENCE PUBLISHING, NEW YORK, NY, US, vol. 202, no. 1, 1 October 2010 (2010-10-01), pages 1-10, XP027246507, ISSN: 0165-4608 [retrieved on 2010-08-27]**
- **SO HYUN SHIN ET AL: "Identification of Novel Methylation Markers in Hepatocellular Carcinoma using a Methylation Array", JOURNAL OF KOREAN MEDICAL SCIENCE, vol. 25, no. 8, 1 January 2010 (2010-01-01), page 1152, XP055163822, ISSN: 1011-8934, DOI: 10.3346/jkms.2010.25.8.1152**
- **RUI-LAN HUANG ET AL: "Integrated Epigenomics Analysis Reveals a DNA Methylation Panel for Endometrial Cancer Detection Using Cervical Scrapings", CLINICAL CANCER RESEARCH, vol. 23, no. 1, 9 August 2016 (2016-08-09), pages 263-272, XP055446410, US ISSN: 1078-0432, DOI: 10.1158/1078-0432.CCR-16-0863**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 301 194 B1

## Description

### TECHNICAL FIELD

The present invention relates to a method for assisting diagnosis of onset risk of gastric cancer in a subject.

### BACKGROUND

The main cause of gastric cancer is Helicobacter pylori infection. When gastric mucosal changes are caused by Helicobacter pylori infection, periodic follow-up observation is recommended for screening of gastric cancer also after sterilization. Gastric cancer examination includes gastroscopy and x-ray examination of the stomach. However, gastroscopy may involve vomiting reflex upon insertion of a gastrocamera, and x-ray examination of the stomach requires drinking barium, thus, in the case of performing examination on a full-year basis, physical and economic burdens to the subject are large.

Recently, methods based on genetic abnormalities accumulated in normal gastric mucosa have been studied as potential methods for diagnosing cancer. Examples of the methods include methods based on information on DNA methylation abnormality. In these methods, a CpG site in a nucleotide sequence of a predetermined gene is used as a marker. Then, based on the analysis result of the methylation status of the marker using gastric mucosal DNA collected by endoscopy or the like information is acquired on the presence or absence of cancer cells, the degree of accumulation of genetic abnormalities and the like, and using this information as index, diagnoses of existence of cancer, pathology of cancer and the like are performed.

For example, US 2016/040244 A discloses that gastric cancer can be diagnosed early by measuring methylation levels of the CpG island of SDC2 gene. In addition, p53 gene, ADCY3 gene, BARHL2 gene, ACGM1 gene, VLDLR gene and the like have been reported as genes highly methylated in gastric cancer (US 2015/254400 A, US 2015/240313 A, JP 2014-161308 A, JP 2007-54059 A, US 2009/054245 A).

### SUMMARY OF THE INVENTION

In order to reduce the burden on subjects who are expected to undergo such periodic examinations, there is a desire to develop a methylation marker that can further evaluate carcinogenic risk so as to allow carcinogenic risk diagnoses using DNA methylation.

The present inventors have focused on the fact that, among the subjects who have experienced Helicobacter pylori infection, there are those who develop and those who do not develop gastric cancer. The present inventors have verified genomic DNA obtained from non-cancerous tissues of gastric cancer patients who experienced a Helicobacter pylori infection and those who experienced a Helicobacter pylori infection but did not develop gastric cancer. Consequently a genetic region in which methylation was found specifically in gastric cancer patients has been identified as a novel marker. Based on the results obtained by analyzing the methylation status of these markers, the present inventors have found that the onset risk of gastric cancer in those who experienced Helicobacter pylori infection can be evaluated. Thereby, the present inventors have completed the present invention.

More specifically, the present invention provides a method for assisting diagnosis of the risk of onset of gastric cancer, including steps of analyzing a methylation status of a CpG site present in a genetic region of the BHLHE22 gene contained in a DNA sample of a subject, and acquiring information on the onset risk of gastric cancer in a subject, based on the result obtained in the analysis step, wherein the DNA sample is prepared from a non-cancerous sample collected from a subject having experienced a Helicobacter pylori infection

### BRIEF DESCRIPTION OF THE DRAWINGS

Fig. 1 are diagrams showing ROC curves in Example 3.
Fig. 2 is a schematic diagram showing an example of a diagnosis assisting apparatus for providing information on the onset risk of gastric cancer in a subject.
Fig. 3 is a block diagram showing a functional configuration of the diagnosis assisting apparatus in Fig. 2.
Fig. 4 is a block diagram showing a hardware configuration of the diagnosis assisting apparatus shown in Fig. 2.
Fig. 5 is a flowchart of determination for providing information on the onset risk of gastric cancer in a subject, using the diagnosis assisting apparatus shown in Fig. 2.

DETAILED DESCRIPTION

In the method for assisting diagnosis of onset risk of gastric cancer described herein (hereinafter, also simply referred to as the "method"), firstly a DNA sample is prepared from a non-cancerous sample collected from a subject having experienced a Helicobacter pylori infection. Subjects having experienced a Helicobacter pylori infection include carriers infected with Helicobacter pylori and those infected in the past but already sterilized. The presence or absence of experience of a Helicobacter pylori infection can be determined by, for example, whether or not atrophic gastritis in the pyloric region is observed by endoscopic observation. Whether or not the subject is a carrier infected with Helicobacter pylori can be determined by, for example, Helicobacter pylori IgG antibody test, urea breath test method or the like. The methods may comprise the step of determining whether or not the subject has experienced a Helicobacter pylori infection.

In the methods envisaged herein, the nature of the non-cancerous sample is not limited as long as it is prepared from a non-cancerous sample collected from a subject. The term "non-cancerous" refers to the fact that it is substantially free from cancer cells. The non-cancerous sample is preferably a sample containing genomic DNA, for example, a clinical specimen. Examples of the clinical specimen include body fluids, urine, tissues collected by a surgery or a biopsy, and the like. Examples of the body fluids include blood, serum, plasma, lymph, ascitic fluid, bone marrow fluid, nipple discharge and the like. A culture obtained by culturing cells or tissues collected from a subject can also be used as the non-cancerous sample. A formalin-fixed paraffin-embedded (FFPE) sample of tissues collected from a subject may be used as the non-cancerous sample. Gastric mucosa substantially free of gastric cancer cells is particularly preferable as the non-cancerous sample.

The DNA sample can be prepared by extracting DNA from the non-cancerous sample. Methods for extracting DNA from the non-cancerous sample are known in the art. For example, DNA can be extracted by mixing the non-cancerous sample with a treatment solution containing a surfactant for lysing cells or tissues (e.g., sodium cholate, sodium dodecyl sulfate, etc.), and subjecting the resulting mixed solution to physical treatment (stirring, homogenizing, ultrasonic disruption, and the like) to release the DNA contained in the non-cancerous sample into the mixed solution. It is preferred that the mixed solution is centrifuged to precipitate cell debris and the supernatant containing released DNA is used in the analysis step described later. The resulting supernatant may be purified by a method known in the art. The extraction and purification of DNA from the non-cancerous sample can also be performed using a commercially available kit.

It is preferred that the preparation step further includes a step of fragmenting the extracted DNA. When the DNA is fragmented into proper lengths, it becomes possible to perform the methylated DNA immunoprecipitation (MeDIP) method and the unmethylated cytosine conversion treatment described later with high efficiency.

The fragmentation of DNA can be performed by ultrasonic treatment, alkali treatment, restriction enzyme treatment, or the like. For example, when the fragmentation of DNA is performed by alkali treatment, the DNA can be fragmented by adding a sodium hydroxide solution to a DNA solution at a final concentration of 0.1 to 1.0 N and incubating the resulting solution at 10 to 40°C for 5 to 15 minutes. When the fragmentation of DNA is performed by restriction enzyme treatment, the restriction enzyme to be used is selected as appropriate based on the nucleotide sequence of the DNA, and for example, MseI, BamHI and the like are used.

In the method described herein, the methylation status of the CpG site present in the genetic region of at least one gene selected from the group consisting of the BHLHE22 gene, FGF12 gene, FLT3 gene, RPRM gene, RIMS1 gene, JAM2 gene, LINC00643 gene, SEPT9 gene, STX16 gene and GUSBP5 gene contained in the DNA obtained in the preparation step is analyzed. The term "genetic region" as used herein may include not only a transcription region but also a region related to the expression control of the gene, such as a promoter region upstream of the transcription start site. The transcription region may include a 5' untranslated region (5' UTR), a gene body (body), a 3' untranslated region (3' UTR), and the like. The gene body may include exons and introns. The term "CpG site" as used herein refers to a site of a sequence in which cytosine (C) and guanine (G) in the nucleotide sequence are arranged adjacently in this order in the 5' to 3' direction. The letter "p" in the CpG represents a phosphodiester bond between cytosine and guanine.

The nucleotide sequences of the BHLHE22 gene, FGF12 gene, FLT3 gene, RPRM gene, RIMS1 gene, JAM2 gene, LINC00643 gene, SEPT9 gene, STX16 gene and GUSBP5 gene themselves are known. These nucleotide sequences are available from known databases such as GeneBank and Ensembl. The accession numbers of Gene bank or Ensembl of the above genes are shown in Table 1.

[Table 1]

| Gene | ACCESSION No. | |
|---|---|---|
| | Genbank | Ensembl |
| BHLHE22 | | ENSG00000180828 |
| FGF12 | | ENSG00000114279 |

(continued)

| Gene | ACCESSION No. | |
|---|---|---|
| | Genbank | Ensembl |
| FLT3 | NG_007066 | ENSG00000122025 |
| RPRM | | ENSG00000177519 |
| RIMS1 | | ENSG00000079841 |
| JAM2 | | ENSG00000154721 |
| LINC00643 (FLJ43390) | NR_015358 | ENSG00000186369 |
| SEPT9 | NG_011683 | ENSG00000184640 |
| STX16 | | ENSG00000124222 |
| GUSBP5 | | ENSG00000236296 |

The gene sequence of the above genes may include, for example, the nucleotide sequences of SEQ ID NOs: 1 to 9 or reverse complementary sequences thereof. SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8 and SEQ ID NO: 9 are regions of BHLHE22, FGF12, FLT3, RPRM, RIMS1, JAM2, LINC00643, SEPT9 and STX16, respectively. In particular, the genetic region of the gene BHLHE22, FGF12, FLT3, RPRM, RIMS1, JAM2, LINC00643, SEPT9and STX16 may be a region comprising SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8 and SEQ ID NO: 9, respectively.

Analysis of the methylation status of the CpG site can be performed, for example, by examining whether or not cytosine in at least one CpG site among the CpG sites present in each genetic region is methylated. The CpG site to be analyzed may be on the Top strand side or on the Bottom strand side. One or multiple CpG sites may be analyzed. Multiple CpG sites may be selected from a genetic region of one gene. Multiple CpG sites may be selected from each of genetic regions of a plurality of genes. Among the CpG sites present in each genetic region, it is preferred to contain the CpG site shown below as an analysis target since it is a CpG site in which methylation specific to a non-cancerous tissue of a gastric cancer patient having experienced a Helicobacter pylori infection is found. Multiple CpG sites contained within the same nucleotide sequence shown below are sites where it has been found that most of the CpG sites are methylated or unmethylated. Therefore, it is unnecessary to detect the methylation of all the CpG sites in the same nucleotide sequence, and the presence or absence of the methylation of at least one CpG site among them is detected, whereby the presence or absence of the methylation of all of the CpG sites can be determined.

1st to 5th CpG sites counted from the 5' end side, among the CpG sites contained in the nucleotide sequence of SEQ ID NO: 1,
1st, 2nd, 26th, 30th and 32nd CpG sites counted from the 5' end side, among the CpG sites contained in the nucleotide sequence of SEQ ID NO: 2,
1st, 18th, 19th, 31st and 72nd CpG sites counted from the 5' end side, among the CpG sites contained in the nucleotide sequence of SEQ ID NO: 3,
1st, 6th, 9th, 11th and 25th CpG sites counted from the 5' end side, among the CpG sites contained in the nucleotide sequence of SEQ ID NO: 4,
1st, 29th, 32nd, 34th and 47th CpG sites counted from the 5' end side, among the CpG sites contained in the nucleotide sequence of SEQ ID NO: 5,
1st, 2nd, 13th, 18th and 29th CpG sites counted from the 5' end side, among the CpG sites contained in the nucleotide sequence of SEQ ID NO: 6,
1st, 13th, 17th, 18th, 20th and 23rd CpG sites counted from the 5' end side, among the CpG sites contained in the nucleotide sequence of SEQ ID NO: 7,
1st, 16th, 17th, 18th and 42nd CpG sites counted from the 5' end side, among the CpG sites contained in the nucleotide sequence of SEQ ID NO: 8, and
1st, 34th and 116th CpG sites counted from the 5' end side, among the CpG sites contained in the nucleotide sequence of SEQ ID NO: 9,.

Accordingly, the genetic region may comprise one or more of the CpG sites indicated above. In particular, the methods described herein may comprise analyzing the methylation status of one or more of the CpG sites of one or more of the genetic regions as listed above.

Methylation analysis may also be performed by examining the frequency of methylation in each genetic region. The phrase "the frequency of methylation" may be, for example, the ratio of the number of methylated CpG sites to the number of CpG sites present in each of the above genetic regions. The analysis target may be the entire genetic region of each of the above genes, or may be a part including at least one CpG site. The analysis target may be determined from a genetic region of any one gene of the above genes. The analysis target may be determined from genetic regions of a plurality of genes. It is preferred that the part including the CpG site described above is to be analyzed.

The frequency of methylation may be a "methylation score" obtained by analyzing the methylation status of the CpG site in the DNA by mass spectrometry such as MassARRAY (registered trademark) described later. In MassARRAY (registered trademark), a DNA fragment is measured, and a methylation score can be calculated from the ratio of the area of a peak derived from a methylated DNA fragment to the area of a peak derived from an unmethylated DNA fragment.

The frequency of methylation in each of the above genetic regions may be calculated manually or using a machine such as a computer.

As the means for methylation analysis, various analysis methods are known in the art. In the methods described herein, the type of analysis method to be employed is not particularly limited. A preferable method is a method including a step of distinguishing methylated DNA from unmethylated DNA, a step of amplifying DNA and a step of detecting the methylated DNA and/or the unmethylated DNA.

Examples of the step of distinguishing methylated DNA from unmethylated DNA include a step of performing a methylation-sensitive restriction enzyme treatment, a MeDIP method, an unmethylated cytosine conversion treatment, or the like.

Examples of the step of amplifying DNA include a step of performing a PCR method, a quantitative PCR method, an IVT (in vitro transcription) amplification method, an SPIA (trademark) amplification method or the like.

Examples of the step of detecting methylated DNA and/or unmethylated DNA include a step of performing an electrophoresis method, a sequence analysis method, a microarray analysis method, mass spectrometry, southern hybridization or the like.

A MeDIP method is a method of concentrating methylated DNA contained in a biological sample, by immunoprecipitation using an anti-methylated cytosine antibody or an anti-methylated cytidine antibody, or an antibody specifically recognizing a methylated DNA binding protein. Methylated DNA contained in the DNA obtained in the extraction step may be concentrated by a MeDIP method, and methylation analysis may be performed on the obtained methylated DNA. Methylated DNA concentrated by the MeDIP method can be amplified by an IVT amplification method or the like, and the obtained amplified product can be subjected to methylation analysis using microarrays. This type of analysis method is called a MeDIP on chip method.

An unmethylated cytosine conversion treatment is a treatment of reacting DNA extracted from a biological sample with an unmethylated cytosine converting agent to convert unmethylated cytosine in the DNA into another base (uracil, thymine, adenine or guanine). The unmethylated cytosine converting agent is a substance capable of reacting with DNA to convert unmethylated cytosine in the DNA into another base (uracil, thymine, adenine or guanine). As the unmethylated cytosine converting agent, for example, bisulfite such as sodium salt, potassium salt, calcium salt or magnesium salt of hydrogen sulfite is suitably used.

In the treatment of DNA using bisulfite, unmethylated cytosine in the DNA is converted to uracil through a deamination reaction, while methylated cytosine does not undergo such base conversion. Therefore, the difference in the methylation status of the CpG site in the DNA results in the difference in a nucleotide sequence (difference between C and U) by unmethylated cytosine conversion treatment using bisulfite. Such an unmethylated cytosine conversion treatment with bisulfite is called bisulfite treatment.

When bisulfite treatment is performed, the amount (concentration) of the bisulfite to be added is not particularly limited, as long as unmethylated cytosine in DNA can be sufficiently converted. The concentration of the bisulfite to be added is, for example, 1 M or more, preferably 1 to 15 M, and more preferably 3 to 10 M, in terms of final concentration in a solution containing the DNA. The conditions (temperature and time) for incubation after the addition of the bisulfite can be set as appropriate depending on the amount of the bisulfite added. For example, when the bisulfite is added at a final concentration of 6 M, the incubation is performed at 50 to 80°C for 10 to 90 minutes.

The methylation of the CpG site contained in the DNA can be analyzed by performing a sequence analysis of bisulfite-treated DNA and detecting a difference from the original nucleotide sequence. This method is called bisulfite sequencing method.

Pyrosequencing method is a method of amplifying bisulfite-treated DNA with PCR and analyzing the PCR product with a pyrosequencer. During PCR, a biotinylated primer (or biotinylated universal primer) is used as a primer on one side to label only one strand of the PCR product with biotin. This is denatured to make it single-stranded, adsorbed with beads and sorted, and then sequence primers are annealed to perform a short sequence. The methylation level of DNA can be analyzed by measuring the ratio of methylated CpG sites.

The methylation of the CpG site can be analyzed by mass spectrometry. Specifically, PCR amplification is performed using a bisulfite-treated DNA as a template and using a primer set specific to a nucleotide sequence to be analyzed, and then the obtained PCR product is further subjected to IVT amplification to convert methylated cytosine and uracil to guanine (G) and adenine (A), respectively. The obtained IVT amplification product is cleaved by RNase A, and the mass difference (16 Da) between G and A among the obtained nucleic acid fragments is detected using a MALDI-TOF (Matrix Assisted Laser Desorption Ionization-Time of Flight) mass spectrometer, whereby DNA methylation can be analyzed. This method is called MassARRAY (registered trademark) analysis.

It is known that the site cleaved by RNase A in the IVT product is a site located between an arbitrary base and uracil (U) or thymine (T) adjacent to the base. Therefore, the nucleotide sequence and mass of the IVT product cleaved by RNase A can be predicted from the nucleotide sequence of the DNA used as a template. Therefore, for each peak obtained with MassARRAY (registered trademark), from which part of the nucleotide sequence of the DNA used as a template can be identified. For example, when one CpG site is methylated in a DNA fragment, a peak obtained in MassARRAY (registered trademark) shifts by 16 Da to the higher mass side. In the analysis of a DNA fragment having multiple CpG sites, for example, the DNA fragment shows a shift by 32 Da when two CpG sites are methylated, and the DNA fragment shows a shift by 48 Da when three CpG sites are methylated in the DNA fragment.

In mass spectrometry such as MassARRAY (registered trademark), the methylation score of the determined DNA fragment can be calculated. For example, when the ratio of the area of a peak for the unmethylated DNA fragment to the area of a peak for the methylated DNA fragment is 1:3 in a chart obtained by the analysis in a DNA fragment of a predetermined sequence, the methylation score of this DNA fragment is 0.75 from the formula $3/(1 + 3) = 0.75$. The methylation score is theoretically 1 when all the CpG sites of the DNA fragment are methylated and 0 when not all the CpG sites are methylated.

The methylation of the CpG site can be analyzed by a methylation specific PCR (MSP) method and a quantitative MSP (qMSP) method. The MSP method is a method of analyzing the presence or absence of methylation of a CpG site by performing PCR amplification of bisulfite-treated DNA using the primer set described later and confirming the presence or absence of a PCR product. In the MSP method, a primer set which can amplify a nucleotide sequence in which a CpG site to be analyzed is methylated (that is, cytosine is not converted to uracil) but cannot amplify a nucleotide sequence in which a CpG site to be analyzed is not methylated (that is, the cytosine is converted to uracil) is used. In the MSP method using the primer set, it is determined that the CpG site to be analyzed is methylated when a PCR product is obtained. The MSP method can also be performed using a primer set which cannot amplify a nucleotide sequence in which cytosine at a CpG site to be analyzed is not converted to uracil but can amplify a nucleotide sequence in which cytosine at a CpG site to be analyzed is converted to uracil. In this case, it is determined that the CpG site to be analyzed is methylated when no PCR product is obtained.

The presence or absence of the PCR product can be confirmed by, for example, a gel electrophoresis method. The amplified reaction solution may be electrophoresed on a gel and whether or not a band derived from the PCR product is present in the gel may be visually confirmed. Alternatively, an image of the gel after electrophoresis may be acquired and confirmed by image analysis. The image analysis may be performed by, for example, acquiring a value indicating the density of pixels (for example, band intensity) in a region in which a PCR product is expected to be present in an image of a gel and comparing this value with a predetermined threshold value. Specifically, when the value indicating the density of the pixels is equal to or greater than the predetermined threshold value, it can be determined that the PCR product has been obtained, and when the value indicating the density of the pixels is less than the predetermined threshold value, the PCR product has not been obtained. The predetermined threshold value relating to the density of the pixels is not particularly limited, and may be, for example, a value indicating the density of the background pixels or may be a value twice or three times that value.

Each of the primers in the primer set used in the MSP method can be designed as appropriate by a person skilled in the art depending on the nucleotide sequence containing the CpG site to be analyzed, and it is preferred that the primer is designed in such a manner that cytosine at the CpG site to be analyzed is contained at the 3' end of the primer or in the vicinity thereof.

In the quantitative MSP method, MSP is performed using real-time PCR procedure, and real-time MSP using SYBR Green I, MethyLight using TaqMan probe and the like are available.

The methylation of the CpG site can also be analyzed using microarrays. In this case, the analytical microarray can be prepared by immobilizing a nucleic acid probe complementary to the nucleotide sequence of the region of each gene on a substrate. The microarray can be prepared by a method known in the art.

In the analysis with microarrays, it is preferred that DNA extracted from the biological sample is labeled with a labeling substance known in the art. Accordingly, it is preferred that the determination method further includes a step of labeling the extracted DNA. It is advantageous that the labeling step is carried out after the above DNA amplification step, because all of the DNAs in the biological sample can be labeled. Examples of the labeling substance include a fluorescent substance, a hapten such as biotin, a radioactive substance, and the like. Examples of the fluorescent substance include Cy3, Cy5, FITC, Alexa Fluor (trademark), and the like. By labeling the DNA as described above, it is easy to measure the signal from the probe on the microarray. The method for labeling the DNA with these labeling substances is known in the art.

The above signal may be an appropriate signal depending on the type of microarray. For example, the signal may be an electrical signal generated when a DNA fragment hybridized with each probe of the microarray is present. In the case where the DNA to be analyzed is labeled as described above, the signal may be a signal such as fluorescence, luminescence, or the like generated from a labeling substance. The signal can be detected by a scanner included in a normal microarray analyzer. Examples of the scanner include GeneChip (registered trademark) Scanner3000 7G (Affymetrix), Illumina (registered trademark) BeadArray Reader (Illumina), and the like.

In the methods described herein, when an analysis result that the methylated CpG site is present is obtained in the above analysis step, information that the subject is in a high risk group of gastric cancer onset (high risk of becoming gastric cancer) can be acquired.

In particular, when a result is obtained that the frequency of methylation obtained by the methylation analysis is higher than or equal to the predetermined threshold value, information that the subject is in a high risk group of gastric cancer onset can be acquired.

The predetermined threshold value is not particularly limited, and can be set empirically by accumulating data on various biological samples. Alternatively, the predetermined threshold value may be set in the following manner. First, the frequency of methylation is analyzed for DNA extracted from each of a biological sample of a healthy person having experience of Helicobacter pylori infection and a non-cancerous sample of a gastric cancer patient having experience of Helicobacter pylori infection. Subsequently, on the basis of the obtained analysis result, a threshold value is set within a range which is higher than the methylation frequency of the biological sample of a healthy person having experienced a Helicobacter pylori infection and is lower than the methylation frequency of the non-cancerous sample of a gastric cancer patient having experienced a Helicobacter pylori infection. Preferably, a value that can distinguish a biological sample of a healthy person having experienced a Helicobacter pylori infection from a non-cancerous sample of a gastric cancer patient having experienced a Helicobacter pylori infection with high accuracy is set as a threshold value.

On the other hand, in the methods described herein, when the analysis result is obtained in the analysis step that the methylated CpG site is not present, information that the subject is in a low risk group of gastric cancer onset (the risk of becoming gastric cancer is low) can be acquired.

In particular, when the frequency of methylation obtained by methylation analysis is lower than the predetermined threshold value, information that the subject is in a low risk group of gastric cancer onset can be acquired.

The present disclosure also includes artificial DNA having a sequence obtained by bisulfite treatment of consecutive nucleotide sequences of all or a part of the genetic regions of each of the above genes. More particularly, disclosed herein are isolated DNA sequences which comprise or consist of a sequence corresponding to all or part of the genetic region of one of the above genes, after bisulfite treatment thereof. More particularly said artificial DNA sequences correspond to all or part of the genetic region of one of the above genes wherein the unmethylated cytosines have been deaminated. More particularly at least one of the cytosines in said region is deaminated. The isolated DNA may correspond to at least 50 nucleotides, more particularly at least 100 or at least 150 nucleotides of said region.The isolated sequence may be less than 1200, more particularly less than 500 nucleotides in length. Most particularly, said region comprises at least one, preferably at least two and in particular embodiments at least all of the CpG sites described herein.The isolated DNA may be artificial DNA, more particularly artificially synthesized DNA.The isolated DNA described herein can be used as a marker for acquiring information on the onset risk of gastric cancer by methylation analysis. Accordingly, it is also envisaged to use the isolated DNA sequences described above in the methods described herein. In such isolated, optionally artificial DNA, the genetic region of each of the above genes may include a CpG site and cytosine not constituting the CpG site. The cytosine not constituting the CpG site may be other than cytosine contained in the CpG site. Examples thereof include cytosine contained in a nucleotide sequence in which cytosine (C) and adenine (A), thymine (T) or cytosine (C) are arranged adjacently in this order in the 5' to 3' direction (that is, CA, CT or CC). Such DNA can be derived from DNA isolated from a subject.

In the uses envisaged herein, methylation analysis is performed on the marker in the DNA sample prepared from a non-cancerous sample collected from a subject having experienced a Helicobacter pylori infection, and based on the obtained analysis result, information on the onset risk of gastric cancer in a subject can be acquired. Methylation analysis and acquisition of information on onset risk of gastric cancer are the same as those described above.

In the isolated and/or artificial DNA used as a marker described herein, unmethylated cytosine in the isolated DNA can be converted to uracil by bisulfite treatment of the isolated DNA, but methylated cytosine can remain as it is. It is possible to detect the presence or absence of methylation and the frequency of methylation by analyzing the nucleotide sequence of the artificial DNA used as such a marker. The above isolated DNA can be obtained in the same manner as the preparation of the DNA sample described above. Bisulfite treatment, methylation analysis and acquisition of information on onset risk of gastric cancer are also the same as those described above.

In the uses envisaged herein, the size of the artificial or isolated DNA used as a marker is not particularly limited as long as it is a size that allows methylation analysis by the MSP method, the qMSP method, the bisulfite sequencing method, the pyrosequencing method or mass spectrometry, and the size is preferably 80 to 500 bases, and more preferably 100 to 400 bases.

Examples of the artificial or isolated DNA envisaged for use as a marker include artificial or isolated DNA consisting of the nucleotide sequences of SEQ ID NOs: 10 to 19. Among them, isolated DNA sequences consisting of the nucleotide sequences of SEQ ID NOs: 11 and 12 are particularly suitable for methylation analysis by the MSP method. Isolated DNA sequences consisting of the nucleotide sequences of SEQ ID NOs: 10, 13 to 19, are particularly suitable for methylation analysis by the pyrosequencing method.

SEQ ID NO: 10, SEQ ID NO: 11, SEQ ID NOs: 12 and 13, SEQ ID NO: 14, SEQ ID NO: 15, SEQ ID NO: 16, SEQ ID NO: 17, SEQ ID NO: 18 and SEQ ID NO: 19 correspond to regions of the BHLHE22 ,FGF12, FLT3, RPRM, RIMS1, JAM2, LINC00643, SEPT9 and STX16 genes, respectively.

Also disclosed herein is a reagent kit for diagnosing onset risk of gastric cancer (hereinafter, also simply referred to as "kit"). The kit may include primers for amplifying bisulfite-treated DNA. Such a primer can hybridize to a nucleic acid including a nucleotide sequence in which cytosine present in a position other than a CpG site has been converted to another base, and hybridize to a region containing a methylated CpG site, in a nucleotide sequence having the CpG site in the genetic region of each gene.

The primers included in the kit can be primers for performing methylation analysis of the CpG site by an analysis method involving PCR amplification such as MSP method, bisulfite sequencing method, pyrosequencing method and the like, and mass spectrometry such as MassARRAY (registered trademark). Preferably, the primers can be a forward primer and a reverse primer used in MSP method, bisulfite sequencing method, pyrosequencing method or mass spectrometry such as MassARRAY (registered trademark). The nucleotide sequence of the primer can be set as appropriate by a person skilled in the art depending on the nucleotide sequence of the genetic region of each of the above genes.The primer sets can be adapted to specifically hybridize to or amplify a sequence within a region of a gene selected from BHLHE22 ,FGF12, FLT3, RPRM, RIMS1, JAM2, LINC00643, SEPT9, STX16 and GUSBP5. Preferably, the region is a region of the genes as specified elsewhere herein. For example, one of the following primer sets can be used.

In the case where the analysis target is BHLHE22 gene, a primer set of SEQ ID NOs: 20 and 21, or SEQ ID NOs: 62 and 63,

in the case where the analysis target is FGF12 gene, a primer set of SEQ ID NOs: 22 and 23,
in the case where the analysis target is FLT3 gene, a primer set of SEQ ID NOs: 24 and 25, or SEQ ID NOs: 38 and 39,
in the case where the analysis target is RPRM gene, a primer set of SEQ ID NOs: 26 and 27,
in the case where the analysis target is RIMS1 gene, a primer set of SEQ ID NOs: 28 and 29,
in the case where the analysis target is JAM2 gene, a primer set of SEQ ID NOs: 30 and 31,
in the case where the analysis target is LINC00643 gene, a primer set of SEQ ID NOs: 32 and 33,
in the case where the analysis target is SEPT9 gene, a primer set of SEQ ID NOs: 34 and 35,
in the case where the analysis target is STX16 gene, a primer set of SEQ ID NOs: 36 and 37, or
in the case where the analysis target is GUSBP5 gene, a primer set of SEQ ID NOs: 64 and 65.

The above kit may contain a second primer. Such a second primer can hybridize to a nucleic acid including a nucleotide sequence in which cytosine has been converted to another base, in a nucleotide sequence having the CpG site in the genetic region of each gene. The second primer can hybridize to a region containing a sequence in which cytosine at the unmethylated CpG site has been converted to another base by conversion treatment. These can easily be determined based on the target gene. For example, in the case where the analysis target is FGF12 gene, a primer set of SEQ ID NOs: 40 and 41 can be used. In the case where the analysis target is FLT3 gene, a primer set of SEQ ID NO: 42 and 43 can be used.

The kit may also include sequence primers for use in pyrosequencing. The nucleotide sequence of the sequence primer can be set as appropriate by a person skilled in the art depending on the nucleotide sequence of the genetic region of each of the above genes. For example, any of the following primers can be used.

In the case where the analysis target is BHLHE22 gene, a primer of SEQ ID NO: 44 or 66,

in the case where the analysis target is RPRM gene, a primer of SEQ ID NO: 45,
in the case where the analysis target is RIMS1 gene, a primer of SEQ ID NO: 46,
in the case where the analysis target is JAM2 gene, a primer of SEQ ID NO: 47,
in the case where the analysis target is LINC00643 gene, a primer of SEQ ID NO: 48,
in the case where the analysis target is SEPT9 gene, a primer of SEQ ID NO: 49,
in the case where the analysis target is STX16 gene, a primer of SEQ ID NO: 50,
in the case where the analysis target is FLT3 gene, a primer of SEQ ID NO: 51, or
in the case where the analysis target is GUSBP5 gene, a primer of SEQ ID NO: 67.

The present disclosure also includes a program product for making a computer acquire information on the onset risk of gastric cancer in a patient. Examples of the program product include a program that can be downloaded via the internet or the like, a computer readable recording medium on which the program is recorded, and the like.

For example, a program for making a computer execute the following steps is exemplified.

A step of acquiring an analysis result of the methylation of the CpG site present in the genetic region of at least one gene selected from the group consisting of the BHLHE22 gene, GUSBP5 gene, RIMS1 gene, LINC00643 gene, FGF12 gene, FLT3 gene, RPRM gene, JAM2 gene, SEPT9 gene and STX16 gene contained in a non-cancerous sample derived from a subject having experience of Helicobacter pylori infection from a measurement device; and
a step of determining onset risk of gastric cancer in the subject, based on the acquired analysis result.

Hereinafter, an example of an apparatus suitable for implementing the method described herein will be described with reference to drawings. However, the present disclosure is not limited to this example. Fig. 2 is a schematic diagram showing an example of a diagnosis assisting apparatus used for acquiring information on the onset risk of gastric cancer in a subject. A diagnosis assisting apparatus 10 shown in Fig. 2 includes a measurement device 20, and a determination device 30 connected to the measurement device 20.

In the apparatus described herein, when methylation analysis is performed by an MSP method, the measurement device 20 may be a gel imaging device such as a fluorescence image scanner. In this case, when a reaction solution subjected to nucleic acid amplification by an MSP method is subjected to gel electrophoresis and the gel after the electrophoresis is set in the measurement device 20, the measuring apparatus 20 detects an amplification product. The measurement device 20 acquires gel image information of the amplification product and provides the obtained information to the determination device 30.

The measurement device 20 may be a MALDI-TOF mass spectrometer. In this case, the measurement device 20 acquires mass spectrometric information such as the flight time and mass-to-charge ratio (m/z value) of the test substance. When a measurement sample prepared from a DNA sample derived from a subject is set in the measurement device 20, the measurement device 20 acquires mass spectrometric information of the nucleic acid contained in the measurement sample and provides the obtained mass spectrometric information to the determination device 30.

The determination device 30 includes a computer main body 300, an input unit 301 including a keyboard and a mouse, and a display unit 302 including an LCD and a CRT and displaying specimen information, a determination result, and the like. The determination device 30 acquires gel image information of the amplification product from the measurement device 20. Based on these pieces of information, the determination device 30 executes a program for providing information on the onset risk of gastric cancer in a subject.

As shown in Fig. 2, the determination device 30 may be an apparatus separate from the measurement device 20, or may be an apparatus including the measurement device 20. In the latter case, the determination device 30 may itself be the diagnosis assisting apparatus 10.

Fig. 3 is a block diagram showing software of the computer main body 300 of the determination device 30 by functional blocks. As shown in Fig. 3, the computer includes an acquisition unit 321, a storage unit 322, a calculation unit 323, a determination unit 324, and an output unit 325. The acquisition unit 321 is communicably connected to the measurement device 20 via a network.

The acquisition unit 321 acquires the information provided from the measurement device 20. The storage unit 322 stores an equation for acquiring a threshold value and band intensity necessary for determination, a processing program, and the like. Using the information acquired by the acquisition unit 321, the calculation unit 323 calculates a band intensity according to the stored processing program. The determination unit 324 determines whether or not the band intensity acquired by the acquisition unit 321 or calculated by the calculation unit 323 is equal to or greater than the threshold value stored in the storage unit 322. The output unit 325 outputs the determination result by the determination unit 324 to the display unit 302 as information on the onset risk of gastric cancer in a subject.

Fig. 4 is a block diagram showing a hardware configuration of the computer main body 300 shown in Fig. 3. As shown in Fig. 4, the computer main body 300 includes a CPU (Central Processing Unit) 310, a ROM (Read Only Memory) 311, a RAM (Random Access Memory) 312, a hard disk 313, an input/output interface 314, a reading device 315, a communication interface 316, and an image output interface 317. The CPU 310, the ROM 311, the RAM 312, the hard disk 313, the input/output interface 314, the reading device 315, the communication interface 316 and the image output interface 317 are data-communicably connected by a bus 318.

The CPU 310 can execute a program stored in the ROM 311 and a program loaded in the RAM 312. By executing the program by the CPU 310, each of the functions shown in Fig. 3 is executed. As a result, the determination device 30 functions as a determination device for providing information on the onset risk of gastric cancer in a subject.

The ROM 311 includes a mask ROM, PROM, EPROM, EEPROM, and the like. In the ROM 311, a program executed by the CPU 310 as described above and data used for the program are recorded.

The RAM 312 includes SRAM, DRAM, and the like. The RAM 312 is used for reading the program recorded in the ROM 311 and the hard disk 313. The RAM 312 is also used as a work area of the CPU 310 when executing these programs.

The hard disk 313 has installed therein an operating system, a program such as an application program (a computer program for providing information on the onset risk of gastric cancer in a subject) to be executed by the CPU 310, and data used for executing the program.

The reading device 315 includes a flexible disk drive, a CD-ROM drive, a DVD-ROM drive, and the like. The reading device 315 can read a program or data recorded in a portable recording medium 40.

The input/output interface 314 includes, for example, a serial interface such as USB, IEEE1394 or RS-232C, a parallel interface such as SCSI, IDE or IEEE1284, and an analog interface including a D/A converter, an A/D converter and the like. The input unit 301 such as a keyboard and a mouse is connected to the input/output interface 314. An operator can input various commands to the computer main body 300 through the input unit 301.

The communication interface 316 is, for example, an Ethernet (registered trademark) interface or the like. The computer main body 300 can also transmit print data to a printer or the like through the communication interface 316.

The image output interface 317 is connected to the display unit 302 including an LCD, a CRT, and the like. As a result, the display unit 302 can output a video signal corresponding to the image data coming from the CPU 310. The display unit 302 displays an image (screen) according to the input video signal.

Next, the processing procedure for acquiring information on the onset risk of gastric cancer in a subject by the diagnosis assisting apparatus 10 will be described.

Fig. 5 is an example of a flow chart for acquiring information on the onset risk of gastric cancer. Here, a case where a band intensity is acquired from the gel image information obtained using a non-cancerous sample derived from a subject having experience of Helicobacter pylori infection, and whether or not the obtained band intensity is equal to or greater than the threshold value is determined will be described as an example. However, the present disclosure is not limited only to this example.

First, in step S1-1, the acquisition unit 321 of the diagnosis assisting apparatus 10 acquires gel image information on the region of each gene from the measurement device 20. Next, in step S1-2, the calculation unit 323 acquires a band intensity from the obtained gel image information, and the calculation unit 323 transmits the band intensity to the storage unit 322.

Thereafter, in step S1-3, the determination unit 324 determines whether or not the band intensity acquired in step S1-2 is equal to or greater than the threshold value stored in the storage unit 322. When the band intensity is less than the threshold value, the routine proceeds to step S1-4. Then, the determination unit 324 transmits a determination result indicating that the subject is in a low risk group of gastric cancer onset to the output unit 325. On the other hand, when the band intensity is equal to or greater than the threshold value, the routine proceeds to step S1-5. Then, the determination unit 324 transmits a determination result indicating that the subject is in a high risk group of gastric cancer onset to the output unit 325.

Finally, in step S1-6, the output unit 325 outputs the determination result as information on the onset risk of gastric cancer in a subject, and the output unit 325 allows the display unit 302 to display the information. As a result, the diagnosis assisting apparatus 10 can provide information that assists in diagnosing whether or not the subject is at high risk of developing gastric cancer to doctors and the like.

Hereinbelow, the present disclosure will be described in detail by way of examples, but the present disclosure is not limited to these examples.

## EXAMPLES

### Example 1: Identification of novel markers based on normal gastric mucosa of healthy person having experience of Helicobacter pylori infection and non-cancerous tissue of gastric cancer patient having experience of Helicobacter pylori infection after endoscopic removal

#### (1) Collection of biological sample

Mucous membranes of one part of the lesser curvature of the antrum were collected from healthy persons having experience of Helicobacter pylori infection (58 specimens; group 2 (G2)). Non-cancerous tissues were collected from gastric cancer patients (96 specimens; group 3 (G3)) having experience of Helicobacter pylori infection after endoscopic removal in the same manner. As a control, mucous membranes were collected from healthy persons (8 specimens; group 1 (G1)) having no experience of Helicobacter pylori infection in the same manner.

#### (2) Preparation of measurement sample

##### (i) Extraction of genomic DNA

Twelve specimens of G2 and twelve specimens of G3 were rinsed and replaced with an aqueous solution containing EDTA and salts having a nucleic acid protective function such as RNAlater in which gastric mucosal tissues were stored, and SDS (final concentration 1%) and proteolytic enzyme (Protease K) were added, then proteolytic treatment was performed overnight at 55°C.
Subsequently, 1 μl of RNase (20 mg/ml) was added, and RNA degradation treatment was performed at 37°C for 1 hour, then protein was removed by phenol · chloroform treatment. Further, DNA was purified by ethanol precipitation and dissolved in 1 × 50 μl of TE solution to prepare genomic DNA.

(ii) Genomic DNA fragmentation

5 μg of the genomic DNA obtained in (i) was subjected to enzyme treatment at 30°C for 15 to 16 hours using 100 units of BamHI (HC: high concentration, TKR1010AH 50 units/μl). After protein removal by phenol · chloroform treatment, DNA was purified by ethanol precipitation and dissolved in 1 × 20 μl of TE solution to obtain a DNA fragment.

(iii) Bisulfite treatment

1 μg of the DNA fragment obtained in (ii) was subjected to bisulfite treatment using innuCONVERT Bisulfite Basic Kit (Analytik jena AG), and the treated DNA fragment was eluted into 1 × 40 μl of TE buffer (10 mM Tris-HCl, 1 mM EDTA).

(3) Infinium analysis

Methylation analysis was performed on DNA fragments after bisulfite conversion using Infinium HumanMethylation450 BeadChip (Illumina). Infinium HumanMethylation450 BeadChip has a probe for methylation and a probe for unmethylation at 482,421 CpG sites among the CpG sites on the human genome. The signal intensity of the probe for methylation (signal M) and the signal intensity of the probe for unmethylation (signal U) of the CpG site of the target gene were detected with the iScan system (illumina), and the methylation ratio of the CpG site (mCpG) of the target gene was calculated by the following calculation formula.

$$(\text{mCpG}) = (\text{signal M})/\{(\text{signal M}) + (\text{signal U})\}$$

Comparing the methylation ratios of G2 and G3, in accordance with the following two methods (conventional method and iEVORA method (Teschendorff, A.E., et al. (2016). "DNA methylation outliers in normal breast tissue identifying field defects that are enriched in cancer." Nat Commun 7: 10478.)), genetic regions of 10 genes in which methylation specific to a non-cancerous tissue of a gastric cancer patient having experience of Helicobacter pylori infection was found were selected as markers.

(Conventional method)

1. Probes on sex chromosomes and probes lacking methylation ratio data are removed from all probes.
2. Among the remaining probes, probes with a methylation ratio of 0.2 or less by blood samples of healthy persons (average of three persons) are extracted.
3. Probes with a methylation ratio of 0.2 or less in G1 are extracted.
4. Probes in which the methylation ratio of G3 is larger than the methylation ratio of G2 by 0.2 or more are extracted.

Probes in which the methylation ratio of G3 is larger than the methylation ratio of G2 by 0.2 or more in the extracted probe and two probes each before and after the extracted probe (continuous five probes) are extracted.

(iEVORA method)

1. Probes on sex chromosomes and probes lacking methylation ratio data are removed from all probes.
2. Probes are extracted (FDR < 0.001, P-value for methylation difference < 0.05 is set as significant), based on the iEVORA algorithm.
GUSBP5 was selected by narrowing down by further adding the following conditions of 3. and 4., in addition to 1.

and 2. of the above iEVORA method.

3. Probes in which the methylation ratio of G3 is larger than the methylation ratio of G2 by 0.2 or more are extracted. Alternatively, probes in which one probe each before and after (continuous three probes) have been extracted in 2. are extracted.

4. Among the remaining probes, probes with a methylation ratio of 0.2 or less by blood samples of healthy persons (average of three persons) are extracted.

The sequence number and gene name of the genetic regions are shown in Table 2. The CpG site where the methylation ratio of G3 has been found to be 0.2 or more higher than the methylation ratio of G2 by Infinium analysis is shown below.

1st to 5th CpG sites counted from the 5' end side, among the CpG sites contained in the nucleotide sequence of SEQ ID NO: 1,

1st, 2nd, 26th, 30th and 32nd CpG sites counted from the 5' end side, among the CpG sites contained in the nucleotide sequence of SEQ ID NO: 2,

1st, 18th, 19th, 31st and 72nd CpG sites counted from the 5' end side, among the CpG sites contained in the nucleotide sequence of SEQ ID NO: 3,

1st, 6th, 9th, 11th and 25th CpG sites counted from the 5' end side, among the CpG sites contained in the nucleotide sequence of SEQ ID NO: 4,

1st, 29th, 32nd, 34th and 47th CpG sites counted from the 5' end side, among the CpG sites contained in the nucleotide sequence of SEQ ID NO: 5,

1st, 2nd, 13th, 18th and 29th CpG sites counted from the 5' end side, among the CpG sites contained in the nucleotide sequence of SEQ ID NO: 6,

1st, 13th, 17th, 18th, 20th and 23rd CpG sites counted from the 5' end side, among the CpG sites contained in the nucleotide sequence of SEQ ID NO: 7,

1st, 16th, 17th, 18th and 42nd CpG sites counted from the 5' end side, among the CpG sites contained in the nucleotide sequence of SEQ ID NO: 8, and

1st, 34th and 116th CpG sites counted from the 5' end side, among the CpG sites contained in the nucleotide sequence of SEQ ID NO: 9,.

Among the CpG sites, the difference between the methylation ratios of G2 and G3, and the ratio of the methylation ratios of G2 and G3 were determined for the CpG sites shown below. The difference in the methylation ratio was defined as a value obtained by subtracting the average value of the methylation ratio of G2 from the average value of the methylation ratio of G3. The ratio of the methylation ratio was defined as a value obtained by dividing the average value of the methylation ratio of G3 by the average value of the methylation ratio of G2. The methylation ratios of G3 and G2 were tested by Welch's t-test. The results are shown in Table 2.

3rd CpG site counted from the 5' end, among the CpG sites contained in the nucleotide sequence of SEQ ID NO: 1,

26th CpG site counted from the 5' end, among the CpG sites contained in the nucleotide sequence of SEQ ID NO: 2,

19th CpG site counted from the 5' end, among the CpG sites contained in the nucleotide sequence of SEQ ID NO: 3,

9th CpG site counted from the 5' end, among the CpG sites contained in the nucleotide sequence of SEQ ID NO: 4,

32nd CpG site counted from the 5' end, among the CpG sites contained in the nucleotide sequence of SEQ ID NO: 5,

13th CpG site counted from the 5' end, among the CpG sites contained in the nucleotide sequence of SEQ ID NO: 6,

17th CpG site counted from the 5' end, among the CpG sites contained in the nucleotide sequence of SEQ ID NO: 7,

17th CpG site counted from the 5' end, among the CpG sites contained in the nucleotide sequence of SEQ ID NO: 8, and

34th CpG site counted from the 5' end, among the CpG sites contained in the nucleotide sequence of SEQ ID NO: 9

[Table 2]

| Sequence number | Gene | UCSC RefGene Group | Relationship with CpG island | Difference in methylation ratio (G3-G2) | Ratio of methylation ratio (G2/G3) | P value (t-test) |
|---|---|---|---|---|---|---|
| 1 | BHLHE22 | TSS200 | N_Shore | 0.289 | 3.54 | 3.37E-04 |
| 2 | FGF12 | TSS200,BODY | Island | 0.227 | 2.30 | 1.49E-04 |
| 3 | FLT3 | TSS200 | Island | 0.327 | 4.30 | 3.97E-07 |
| 4 | RPRM | TSS1500 | S_Shore | 0.285 | 2.91 | 1.18E-04 |
| 5 | RIMS1 | TSS200 | S_Shore | 0.347 | 3.33 | 4.22E-04 |
| 6 | JAM2 | 1stExon;5'UTR | Island | 0.29 | 2.95 | 1.28E-04 |
| 7 | LINC00643 | TSS200 | Island | 0.238 | 2.43 | 6.62E-05 |
| 8 | SEPT9 | TSS200;5'UTR; Body | Island | 0.161 | 6.571 | 8.01E-03 |
| 9 | STX16 | TSS1500 | Island | 0.245 | 5.122 | 1.29E-04 |
| | GUSBP5 | TSS200 | Island | | | |
| *TSS200 or 1500 means a region of 200 bases or 1500 bases upstream of the transcription start site. | | | | | | |

Example 2:

The genetic regions of 10 genes selected in Example 1 was verified using MSP or pyrosequencing method.

(1) For 12 specimens of G2 and 12 specimens of G3 that are different specimens collected from the same group as the specimen used in Example 1, measurement samples were prepared in the same manner as in Example 1.
(2) Quantitative methylation specific PCR (qMSP)

Methylation analysis was performed on FLT3 gene and FGF12 gene by qMSP, using the measurement sample obtained in (1) above. The compositions of the PCR reagents used, primer sets, PCR conditions and CpG sites to be analyzed are shown below. qMSP was performed by real-time PCR using SYBR Green I (BioWhittaker Molecular Applications) and CFX connect (Bio-Rad Laboratories). The number of methylated molecules (M) and the number of unmethylated molecules (U) were measured, and the methylation ratio was calculated by the following calculation formula. The difference in the methylation ratio was defined as a value obtained by subtracting the average value of the methylation ratio of G2 from the average value of the methylation ratio of G3. The ratio of the methylation ratio was defined as a value obtained by dividing the average value of the methylation ratio of G3 by the average value of the methylation ratio of G2. The methylation ratios of G3 and G2 were tested by Welch's t-test. The results are shown in Table 4.

$$\text{Methylation ratio} = M/(M + U)$$

<PCR reagent>

DW (sterile water) 14.3 μL

(continued)

| | |
|---|---|
| 10X PCR buffer ($MgCl_2$ 15 mM) | 2.0 μL |
| dNTP (2 mM) | 2.0 μL |
| 10 × SYBR Green I solution | 0.1 μL |
| AmpliTaq Gold (5U/μl) | 0.2 μL |
| 20 μM Forward primer | 0.2 μL |
| 20 μM Reverse primer | 0.2 μL |
| Measurement sample | 1 μL |
| Total | 20 μL |

<PCR reaction conditions>

Annealing temperature: FGF12 (M 62°C, U 62°C), FLT3 (M 62°C, U 64°C)
PCR program: 95°C (15 min) - [94°C (30 sec) - Annealing temperature (30 sec) - 72°C (30 sec)] × 40 cycle - 72°C (10 min) -15°C (∞)

<Primer set>

The primer sets used in the qMSP are shown in Table 3. In the table, "M" is a primer set capable of obtaining an amplification product when the DNA of the region to be amplified is methylated (hereinafter, also referred to as "primer set for detecting methylation"), and "UM" is a primer set capable of obtaining an amplification product when the DNA of the region to be amplified is not methylated (hereinafter, also referred to as "primer set for detecting unmethylation"). In each genetic region, the nucleotide sequences of the regions analyzed by the primer set for detecting methylation were indicated by SEQ ID NOs: 53 (FGF12) and 54 (FLT3).

In the primer sets of SEQ ID NOs: 22 and 23, and SEQ ID NOs: 40 and 41, 24th to 28th CpG sites counted from the 5' end of the nucleotide sequence of SEQ ID NO: 2 are to be analyzed. In the primer sets of SEQ ID NOs: 24 and 25, and SEQ ID NOs: 42 and 43, 18th to 20th and 29th to 30th CpG sites counted from the 5' end of the nucleotide sequence of SEQ ID NO: 3 are to be analyzed.

<CpG site to be analyzed>

26th CpG site counted from the 5' end, among the CpG sites contained in the nucleotide sequence of SEQ ID NO: 2, and
19th CpG site counted from the 5' end, among the CpG sites contained in the nucleotide sequence of SEQ ID NO: 3.

[Table 3]

| Gene | | Sequence number | Forward | Sequence number | Reverse | Amplicon size |
|---|---|---|---|---|---|---|
| FGF12 | M | 22 | TTGGAGAGGATATTTTCGGTATC | 23 | ACTTTCCGACACGAAACG | 119 |
| | UM | 40 | GAGTTGGAGAGGATATTTTTGGTATT | 41 | CTTTCCAACACAAAACAAACA | 121 |
| FLT3 | M | 24 | GTTCGGTTGTAGCGTTGC | 25 | ACTCTCTTAAAAATACGCGTCACCG | 159 |
| | UM | 42 | TGTGGGTTTGGTTGTAGTGTTGT | 43 | ACTCTCTTAAAAATACACATCACCAACCA | 164 |

[Table 4]

| Gene | Difference in methylation ratio (G3-G2) | Ratio of methylation ratio (G2/G3) | P value (T-test) |
|---|---|---|---|
| FGF12 | 0.293 | 2.19 | 2.70E-02 |
| FLT3 | 0.159 | 4.99 | 7.11E-03 |

(3) Pyrosequencing

The DNA of the measurement sample was amplified using the primer set described in Table 5. The compositions of the PCR reagents used, primer sets, PCR conditions and CpG sites to be analyzed are shown below. Pyrosequencing was performed using PSQ 96 Pyrosequencing System (Qiagen). The methylation ratio was calculated using PSQ Assay Design software (Qiagen). The difference in the methylation ratio was defined as a value obtained by subtracting the average value of the methylation ratio of G2 from the average value of the methylation ratio of G3. The ratio of the methylation ratio was defined as a value obtained by dividing the average value of the methylation ratio of G3 by the average value of the methylation ratio of G2. The methylation ratios of G3 and G2 were tested by Welch's t-test. The results are shown in Table 6.

<PCR reagent>

| | |
|---|---|
| DW (sterile water) | 15 μL |
| 2xPyroMark PCR Mix | 22 μL |
| 10xCoralLord Conc. | 4.4 μL |
| 25 mM $MgCl_2$ | 0.4 μL |
| 5 μM Forward primer | 1.1 μL |
| 5 μM Reverse primer | 1.1 μL |
| Measurement sample | 1 μL |
| Total | 45 μL |

<PCR reaction conditions>

Annealing temperature: BHLHE22(1): 55°C, BHLHE22(2): 51°C, RPRM: 50°C, RIMS1: 57°C, JAM2: 58°C, LINC00643: 52°C, STX16: 56°C, FLT3: 58°C, GUSBP5: 56°C PCR program: 95°C (15 min) - [94°C (30 sec) - Annealing temperature (30 sec) - 72°C (30 sec)] × 40 cycle - 72°C (10 min) -15°C (∞)

<Primer>

The primer sets for PCR amplification used in the above-described pyrosequencing and sequence primers for pyrosequencing are shown in Table 5. The nucleotide sequences of the regions analyzed by each primer set are shown by SEQ ID NOs: 52, 55 to 61.

<CpG site to be analyzed>

3rd CpG site counted from the 5' end, among the CpG sites contained in the nucleotide sequence of SEQ ID NO: 1,

19th CpG site counted from the 5' end, among the CpG sites contained in the nucleotide sequence of SEQ ID NO: 3,
9th CpG site counted from the 5' end, among the CpG sites contained in the nucleotide sequence of SEQ ID NO: 4,
32nd CpG site counted from the 5' end, among the CpG sites contained in the nucleotide sequence of SEQ ID NO: 5,
13th CpG site counted from the 5' end, among the CpG sites contained in the nucleotide sequence of SEQ ID NO: 6,
17th CpG site counted from the 5' end, among the CpG sites contained in the nucleotide sequence of SEQ ID NO: 7,
17th CpG site counted from the 5' end, among the CpG sites contained in the nucleotide sequence of SEQ ID NO: 8, and

34th CpG site counted from the 5' end, among the CpG sites contained in the nucleotide sequence of SEQ ID NO: 9

[Table 5]

| Gene | Sequence number | Forward primer | Sequence number | Reverse primer | Amplicon size | Sequence number | Sequence primer | Sequence number of nucleotide sequence of region to be analyzed |
|---|---|---|---|---|---|---|---|---|
| BHLHE22(1) | 20 | AGGGTAATTTAGTATA AGTTGAGATAGTAGTG* | 21 | CTCCATTATTCTACC TACCATCTATATACAC | 298 | 44 | ATTATTCTACCTAC CATCTA | 52 |
| BHLHE 22(2) | 62 | TTATTTTTTAGGTTTGA AGATAGGTAGTAGT* | 63 | CCTCCACTCTAAATAAAA CTTTTAATATAC | 210 | 66 | CAACCCCTTACAA ACAT | Sequence obtained by bisulfite treatment of reverse complement sequence of SEQ ID NO: 1 |
| RPRM | 26 | GTGGTTTTAGTAGGTY TTATTTGGTATTT | 27 | RAACCTAATCTTACTTTTT ATTACTTACTTC* | 247 | 45 | GGGGTAGTAGGTA AAGGAT | 56 |
| RIMS1 | 28 | AATTGGAGGTAGTA GAGTTTGGGAGTA* | 29 | CCCAATCTTTCATAATTAT TTACATCC | 299 | 46 | CCCCRAATACTAC CTTT | 57 |
| JAM2 | 30 | GATTCGTTTTTTGGTA GTTAGTTGAGAA | 31 | TAAAAAAACCCGAACAA CTTACAAC* | 290 | 47 | TTTTGGTAGTTAG TTGAGAA | 58 |
| LINC00643 | 32 | GGGGATATRGAGTTAA GGTTTGTAG* | 33 | ACAACTCYAAAACAACT TTCCTATAA | 257 | 48 | TCCAAAACAACT TTCCTAT | 59 |
| SEPT9 | 34 | TTAGAGTGGTAGTTGG GGGATAGTTT | 35 | AAAAAAAAATCTCTTCCC CTCTAAAC* | | 49 | GGGGATAGTTTTG GGTA | 60 |
| STX16 | 36 | GGAAAATATATACGTG TGTAAAGTTTG | 37 | AAAAACTTAAAACCAAA AACCTAAC* | | 50 | AAGGAAGAGTGG GGG | 61 |
| FLT3 | 38 | AGTTCGGGGATTTATA GGGGTAGTA* | 39 | CGCGCTACAAATAACTCT CTTAAAAATA | | 51 | CCAACAACCCATA CCTAA | 55 |

(continued)

| Gene | Sequence number | Forward primer | Sequence number | Reverse primer | Amplicon size | Sequence number | Sequence primer | Sequence number of nucleotide sequence of region to be analyzed |
|---|---|---|---|---|---|---|---|---|
| GUSBP5 | 64 | TATTAGTAGAGAGGGAGGAGTTTTG* | 65 | CCCAACAATAATACTAAAAAATCAAAATCT | 228 | 67 | TTTAAAAAAATACTTTCCCT | 70 |

* is a biotin-labeled primer. In the nucleotide sequence. R represents A or G. and Y represents C or T.
BHLHE22(2) targets the reverse complementary sequence of the target sequence of BHLHE22(1). but the CpG site to be analyzed is the same.

[Table 6]

| Gene | Difference in methylation ratio (G3-G2) | Ratio of methylation ratio (G2/G3) | P value (t-test) |
|---|---|---|---|
| BHLHE22(1) | 0.182 | 2.09 | 1.11 E-03 |
| RPRM | 0.234 | 3.09 | 7.89 E-04 |
| RIMS1 | 0.205 | 2.70 | 1.39 E-04 |
| JAM2 | 0.265 | 3.18 | 6.23 E-04 |
| LINC00643 | 0.119 | 2.56 | 2.20 E-03 |
| FLT3 | 0.211 | 4.14 | 3.59 E-04 |
| GUSBP5 | 0.108 | 1.99 | 5.8E-03 |

Example 3: Validation

(1) For 22 specimens of G2 and 41 specimens of G3 different from the specimens used in Examples 1 and 2, measurement samples were prepared in the same manner as in Example 1, and then qMSP and pyrosequencing were performed in the same manner as in Example 2. The results are shown in Table 7.

[Table 7]

| Gene | Difference in methylation ratio (G3-G2) | Ratio of methylation ratio (G2/G3) | P value (t-test) |
|---|---|---|---|
| BHLHE22(1) | 0.203 | 2.39 | 6.52 E-05 |
| BHLHE22(2) | 0.143 | 2.49 | 1.03E-03 |
| FGF12 | 0.384 | 3.21 | 1.67 E-04 |
| FLT3(MSP) | 0.179 | 5.44 | 8.76 E-05 |
| RPRM | 0.178 | 2.37 | 2.93 E-04 |
| RIMS1 | 0.154 | 2.15 | 9.48 E-05 |
| JAM2 | 0.126 | 1.89 | 2.41 E-03 |
| LINC00643 | 0.113 | 2.05 | 3.91 E-04 |
| FLT3(pyro) | 0.141 | 3.03 | 1.22E-05 |
| GUSBP5 | 0.127 | 2.28 | 5.61E-05 |

(2) For each genetic region and miR124a-3, ROC analysis was performed. The results are shown in Table 8. The ROC curve is shown in Fig 1

[Table 8]

| | miR124 a-3 | BHLHE22 (1) | BHLHE22 (2) | FGF12 | FLT3 (MSP) | FLT3 (pyro) | RPRM | RIMS1 | JAM2 | LINC 00643 | GUS BP5 |
|---|---|---|---|---|---|---|---|---|---|---|---|
| Cutoff point | 22.8 | 14.7 | 9.13 | 30.4 | 9.3 | 18.59 | 16.4 | 18.7 | 20.0 | 13.0 | 20.2 |
| True positive (TP) | 31 | 34 | 33 | 27 | 27 | 24 | 29 | 31 | 28 | 29 | 21 |
| False positives (FP) | 5 | 6 | 5 | 3 | 1 | 2 | 4 | 5 | 3 | 5 | 1 |
| False negative (FN) | 10 | 7 | 8 | 13 | 14 | 17 | 12 | 10 | 13 | 12 | 20 |
| True negative (TN) | 17 | 16 | 17 | 17 | 21 | 20 | 18 | 17 | 19 | 17 | 21 |
| True positive rate (TPR) | 0.76 | 0.83 | 0.80 | 0.68 | 0.66 | 0.59 | 0.71 | 0.76 | 0.68 | 0.71 | 0.51 |
| False positive rate (FPR) | 0.23 | 0.27 | 0.23 | 0.15 | 0.05 | 0.09 | 0.18 | 0.23 | 0.14 | 0.23 | 0.05 |
| Difference between true positive rate and false positive rate (TPR-FPR) | 0.53 | 0.56 | 0.578 | 0.53 | 0.61 | 0.49 | 0.53 | 0.53 | 0.55 | 0.48 | 0.47 |
| (Youden index) | | | | | | | | | | | |
| Sensitivity | 0.76 | 0.76 | 0.8 | 0.63 | 0.66 | 0.59 | 0.71 | 0.71 | 0.83 | 0.68 | 0.51 |
| Specificity | 0.77 | 0.77 | 0.77 | 0.86 | 0.95 | 0.91 | 0.77 | 0.82 | 0.73 | 0.86 | 0.95 |
| Area under ROC curve (AUC) | 0.75 | 0.78 | 0.78 | 0.77 | 0.80 | 0.79 | 0.76 | 0.76 | 0.74 | 0.76 | 0.76 |
| Odds ratio (OR) | 10.5 | 13.0 | 14.0 | 11.8 | 40.5 | 14.1 | 10.9 | 10.5 | 13.6 | 8.2 | 22.1 |

In each of the above genetic regions, a statistically significant difference was observed in the methylation ratio between G3 and G2 and was also reproduced in the validation cohort. Also in the ROC analysis, these markers showed sensitivity and specificity equal to or higher than those of miR124a-3 which was conventionally used.

## Claims

**1.** A method for assisting diagnosis of onset risk of gastric cancer in a subject, comprising steps of
analyzing a methylation status of a CpG site present in a genetic region of the BHLHE22 gene contained in a DNA sample of the subject, and
obtaining information on the onset risk of gastric cancer in the subject, based on the result obtained in the analysis step,
wherein the DNA sample is prepared from a non-cancerous sample collected from the subject, the subject having experienced a Helicobacter pylori infection.

**2.** The method according to claim 1, wherein the analysis step is a step of analyzing the presence or absence of methylation of a CpG site.

3. The method according to claim 1 or 2, wherein when the analysis result is obtained that methylation is present in the analysis step, information that the subject is in a high risk group of gastric cancer onset is acquired in the information acquisition step.

4. The method according to any of claims 1 to 3, wherein when the analysis result is obtained that methylation is not present in the analysis step, information that the subject is in a low risk group of gastric cancer onset is acquired in the information acquisition step.

5. The method according to claim 1, wherein the analysis step is a step of analyzing the frequency of methylation.

6. The method according to claim 5, wherein, when the frequency of methylation is higher than a predetermined threshold value in the analysis step, information that the patient is in a high risk group of gastric cancer onset in the information acquisition step is acquired.

7. The method according to claim 5 or 6, wherein, when the frequency of methylation is lower than a predetermined threshold value in the analysis step, information that the patient is in a low risk group of gastric cancer onset in the information acquisition step is acquired.

8. The method according to any of claims 1 to 7, wherein the non-cancerous sample is gastric mucosa substantially free of gastric cancer cells.

9. The method according to any of claims 1 to 8, wherein the genetic region of the BHLHE22 gene comprises a nucleotide sequence of SEQ ID NO: 1.

10. The method according to any of claims 1 to 9, wherein in the analysis step, the CpG site for analyzing the methylation status is at least one selected from the 1st to 5th CpG sites counted from the 5' end side, among the CpG sites contained in the nucleotide sequence of SEQ ID NO: 1.

11. The method according to claims 1 to 10, wherein the analysis of the methylation status is performed by at least one selected from microarray, sequencing, mass spectrometry and methylation specific PCR.

**Patentansprüche**

1. Verfahren zur Unterstützung der Diagnose des Risikos zur Entstehung von Magenkrebs bei einem Individuum, umfassend die Schritte des
Analysierens eines Methylierungszustands einer CpG-Stelle, die in einer genetischen Region des BHLHE22-Gens vorhanden ist, die in einer DNA-Probe des Individuums enthalten ist, und
Erhalten von Informationen bezüglich des Risikos zur Entstehung von Magenkrebs bei dem Individuum, basierend auf dem in dem Analyseschritt erhaltenen Ergebnis,
wobei die DNA-Probe aus einer nicht kanzerösen Probe hergestellt wird, die dem Individuum entnommen wurde, wobei das Individuum eine *Helicobacter-pylori-Infektion* durchlebt hat.

2. Verfahren nach Anspruch 1, wobei der Analyseschritt ein Schritt des Analysierens des Vorhandenseins bzw. der Abwesenheit von Methylierung einer CpG-Stelle ist.

3. Verfahren nach Anspruch 1 oder 2, wobei, wenn im Analyseschritt das Analyseergebnis erhalten wird, dass Methylierung vorhanden ist, im Informationsgewinnungsschritt die Information gewonnen wird, dass das Individuum zu einer Hochrisikogruppe für die Entstehung von Magenkrebs gehört.

4. Verfahren nach einem der Ansprüche 1 bis 3, wobei, wenn im Analyseschritt das Analyseergebnis erhalten wird, dass keine Methylierung vorhanden ist, im Informationsgewinnungsschritt die Information gewonnen wird, dass das Individuum zu einer Niedrigrisikogruppe für die Entstehung von Magenkrebs gehört.

5. Verfahren nach Anspruch 1, wobei der Analyseschritt ein Schritt des Analysierens der Methylierungshäufigkeit ist.

6. Verfahren nach Anspruch 5, wobei, wenn im Analyseschritt die Methylierungshäufigkeit höher als ein zuvor bestimmter Schwellenwert ist, im Informationsgewinnungsschritt die Information gewonnen wird, dass das Individuum zu einer Hochrisikogruppe für die Entstehung von Magenkrebs gehört.

**7.** Verfahren nach Anspruch 5 oder 6, wobei, wenn im Analyseschritt die Methylierungshäufigkeit niedriger als ein zuvor bestimmter Schwellenwert ist, im Informationsgewinnungsschritt die Information gewonnen wird, dass das Individuum zu einer Niedrigrisikogruppe für die Entstehung von Magenkrebs gehört.

**8.** Verfahren nach einem der Ansprüche 1 bis 7, wobei es sich bei der nicht kanzerösen Probe um Magenschleimhaut handelt, die im Wesentlichen frei von Magenkrebszellen ist.

**9.** Verfahren nach einem der Ansprüche 1 bis 8, wobei die genetische Region des BHLHE22-Gens eine Nukleotidsequenz von SEQ ID NO: 1 umfasst.

**10.** Verfahren nach einem der Ansprüche 1 bis 9, wobei im Analyseschritt die CpG-Stelle zum Analysieren des Methylierungszustands wenigstens eine ausgewählt aus den 1. bis 5. CpG-Stellen gezählt vom 5'-Ende, unter den CpG-Stellen, die in der Nukleotidsequenz von SEQ ID NO: 1 enthalten sind, ist.

**11.** Verfahren nach den Ansprüchen 1 bis 10, wobei die Analyse des Methylierungszustands durch wenigstens eines ausgewählt aus Mikroarray, Sequenzierung, Massenspektrometrie und methylierungsspezifische PCR durchgeführt wird.

## Revendications

**1.** Procédé d'assistance au diagnostic du risque d'apparition du cancer gastrique chez un sujet, comprenant les étapes de
analyse d'un statut de méthylation d'un site CpG présent dans une région génétique du gène BHLHE22 contenu dans un échantillon d'ADN du sujet, et
obtention d'une information sur le risque d'apparition du cancer gastrique chez le sujet, sur la base du résultat obtenu dans l'étape d'analyse,
dans lequel l'échantillon d'ADN est préparé à partir d'un échantillon non-cancéreux prélevé du sujet, le sujet ayant subi une infection à Helicobacter pylori.

**2.** Procédé selon la revendication 1, dans lequel l'étape d'analyse est une étape d'analyse de la présence ou de l'absence de méthylation d'un site CpG.

**3.** Procédé selon la revendication 1 ou 2, dans lequel, quand le résultat de l'analyse tel qu'obtenu est qu'une méthylation est présente dans l'étape d'analyse, l'information selon laquelle le sujet entre dans un groupe à haut risque d'apparition du cancer gastrique est acquise dans l'étape d'acquisition de l'information.

**4.** Procédé selon l'une quelconque des revendications 1 à 3, dans lequel, quand le résultat de l'analyse tel qu'obtenu est qu'une méthylation n'est pas présente dans l'étape d'analyse, l'information selon laquelle le sujet entre dans un groupe à faible risque d'apparition du cancer gastrique est acquise dans l'étape d'acquisition de l'information.

**5.** Procédé selon la revendication 1, dans lequel l'étape d'analyse est une étape d'analyse de la fréquence de méthylation.

**6.** Procédé selon la revendication 5, dans lequel, quand la fréquence de méthylation est supérieure à une valeur seuil prédéterminée dans l'étape d'analyse, l'information selon laquelle le patient entre dans un groupe à haut risque d'apparition du cancer gastrique dans l'étape d'acquisition de l'information est acquise.

**7.** Procédé selon la revendication 5 ou 6, dans lequel, quand la fréquence de méthylation est inférieure à une valeur seuil prédéterminée dans l'étape d'analyse, l'information selon laquelle le patient entre dans un groupe à faible risque d'apparition du cancer gastrique dans l'étape d'acquisition de l'information est acquise.

**8.** Procédé selon l'une quelconque des revendications 1 à 7, dans lequel l'échantillon non-cancéreux est une muqueuse gastrique sensiblement exempte de cellules de cancer gastrique.

**9.** Procédé selon l'une quelconque des revendications 1 à 8, dans lequel la région génétique du gène BHLHE22 comprend une séquence nucléotidique de SEQ ID NO: 1.

**10.** Procédé selon l'une quelconque des revendications 1 à 9, dans lequel, dans l'étape d'analyse, le site CpG pour l'analyse du statut de méthylation est au moins un site choisi parmi le 1er au 5ème sites CpG, comptés à partir de l'extrémité 5', parmi les sites CpG contenus dans la séquence nucléotidique de SEQ ID NO: 1.

**11.** Procédé selon les revendications 1 à 10, dans lequel l'analyse du statut de méthylation est réalisée par au moins une technique choisie parmi une puce à ADN, un séquençage, une spectrométrie de masse et une PCR spécifique de méthylation.

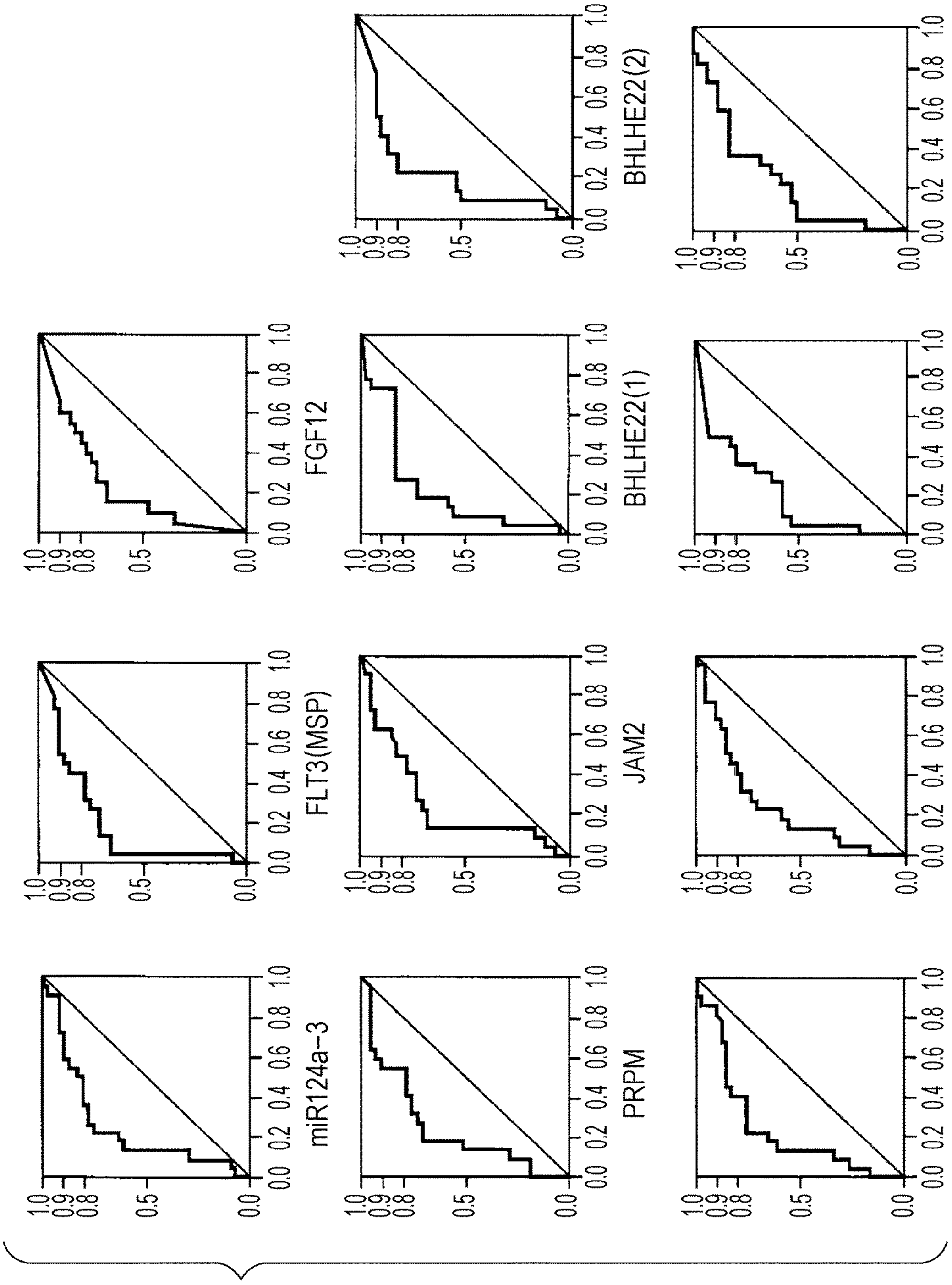

miR124a−3
FLT3(MSP)
FGF12
PRPM
JAM2
BHLHE22(1)
BHLHE22(2)
RIMS1
LINC00643
FLT3(pyro)
GUSBP5


FIG. 1

FIG. 2

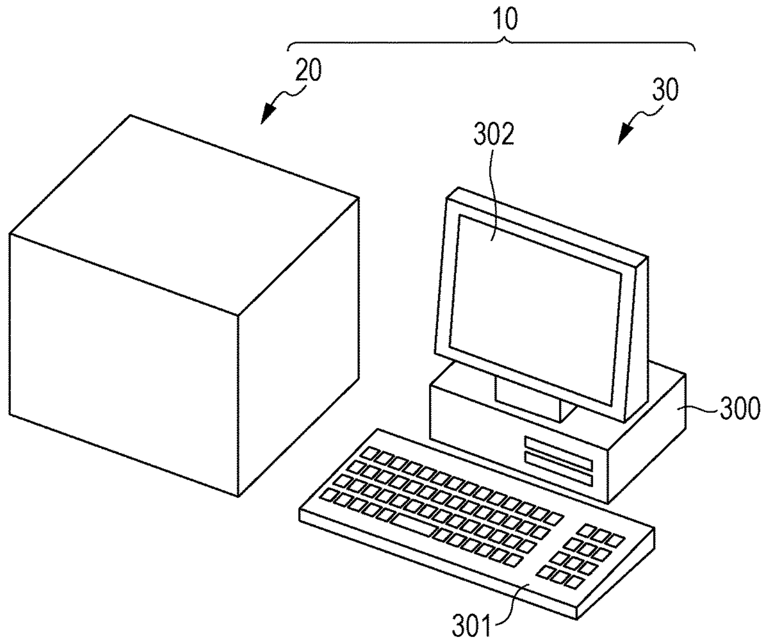
10
20
30
302
300
301

FIG. 3

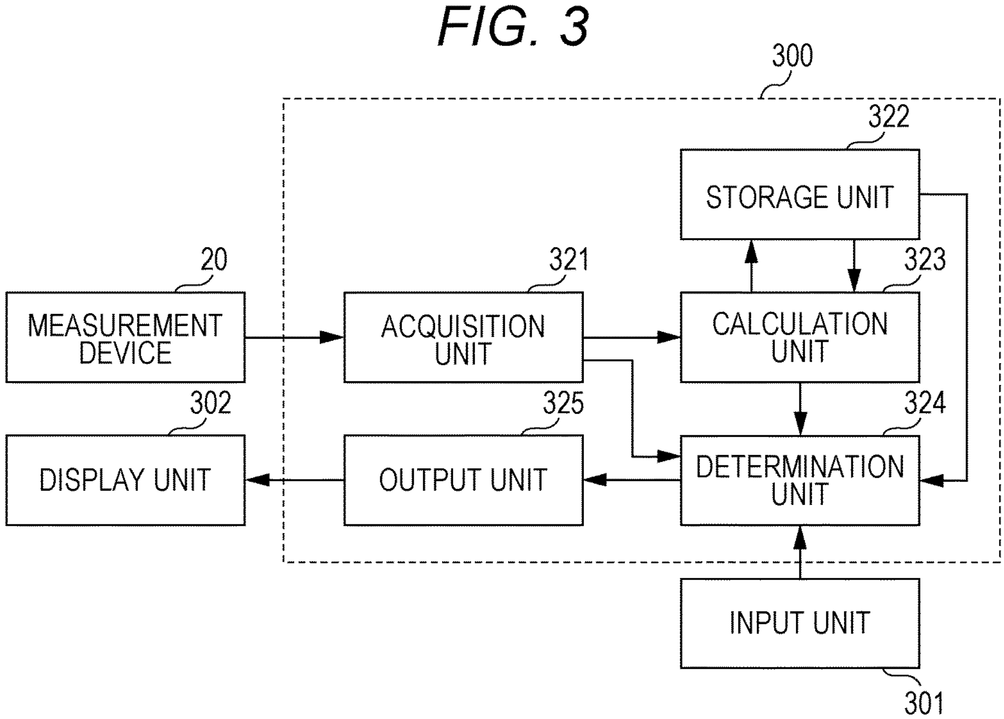
300
322
STORAGE UNIT
20
321
323
MEASUREMENT DEVICE
ACQUISITION UNIT
CALCULATION UNIT
302
325
324
DISPLAY UNIT
OUTPUT UNIT
DETERMINATION UNIT
INPUT UNIT
301

FIG. 4

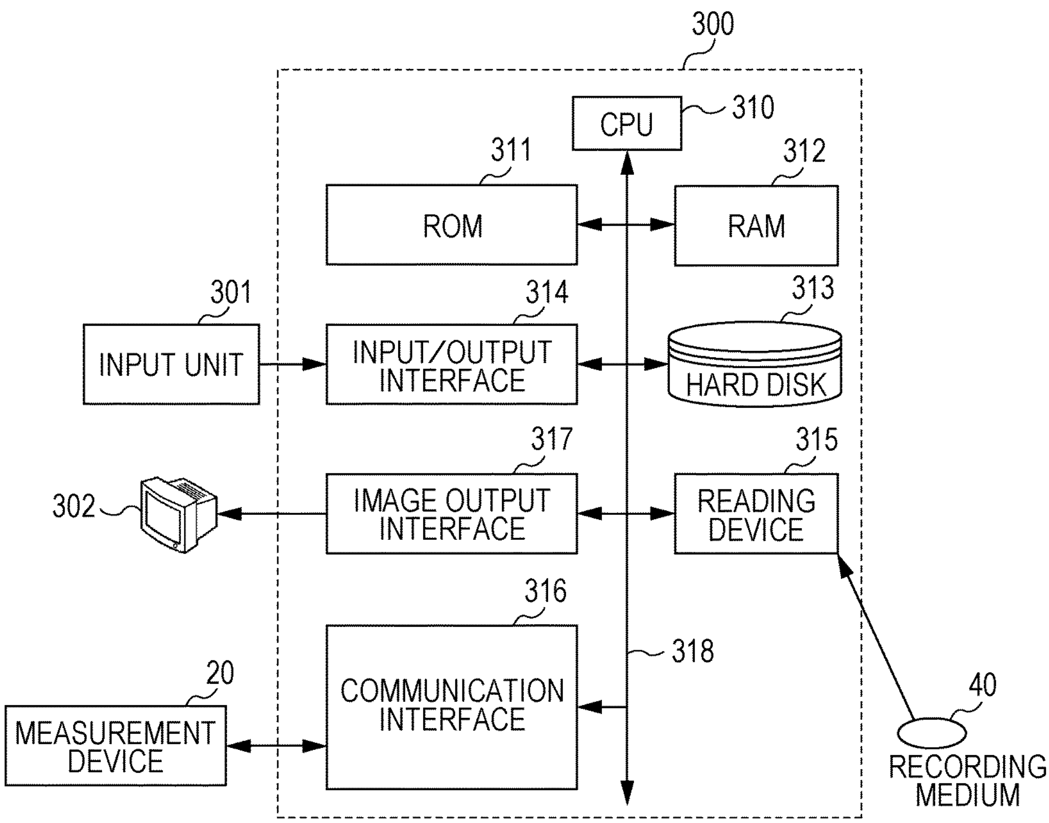
300
CPU
310
311
ROM
312
RAM
301
INPUT UNIT
314
INPUT/OUTPUT INTERFACE
313
HARD DISK
317
302
IMAGE OUTPUT INTERFACE
315
READING DEVICE
316
318
20
MEASUREMENT DEVICE
COMMUNICATION INTERFACE
40
RECORDING MEDIUM

FIG. 5

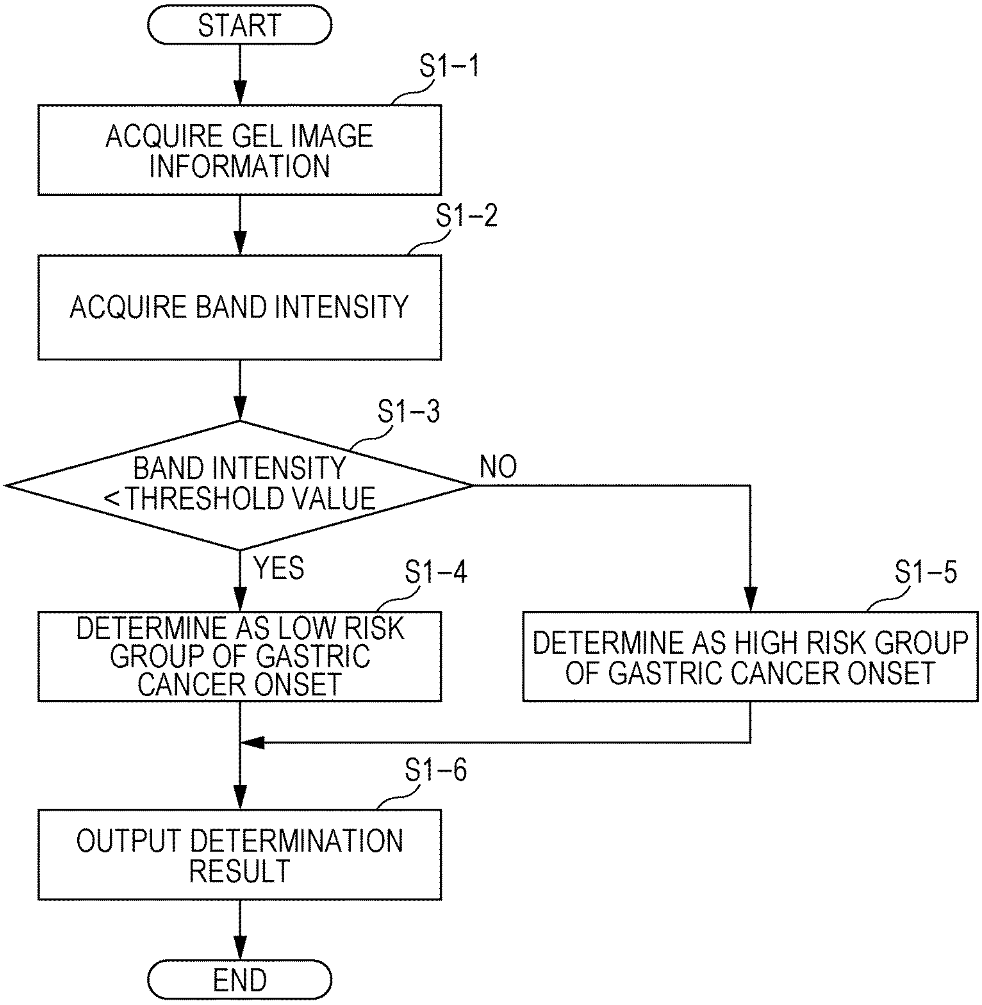
START
S1-1
ACQUIRE GEL IMAGE INFORMATION
S1-2
ACQUIRE BAND INTENSITY
S1-3
BAND INTENSITY <THRESHOLD VALUE
NO
YES
S1-4
DETERMINE AS LOW RISK GROUP OF GASTRIC CANCER ONSET
S1-5
DETERMINE AS HIGH RISK GROUP OF GASTRIC CANCER ONSET
S1-6
OUTPUT DETERMINATION RESULT
END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2016040244 A
- US 2015254400 A
- US 2015240313 A
- JP 2014161308 A
- JP 2007054059 A
- US 2009054245 A
- JP 2016192971 A
- JP 2017140546 A


### Non-patent literature cited in the description


- **TESCHENDORFF, A.E. et al.** DNA methylation outliers in normal breast tissue identifying field defects that are enriched in cancer. *Nat Commun,* 2016, vol. 7, 10478

SEQUENCE LISTING

<110> National Cancer Center
SYSMEX CORPORATION

<120> METHOD FOR ASSISTING DIAGNOSIS OF ONSET RISK OF GASTRIC CANCER, AND ARTIFICIAL DNA AND KIT FOR DIAGNOSING ONSET RISK OF GASTRIC CANCER USED IN THE METHOD

<130> SYSM-125-EP

<150> JP2016-192971
<151> 20160930

<150> JP2017-140546
<151> 20170720

<160> 61

<170> PatentIn version 3.5

<210> 1
<211> 179
<212> DNA
<213> Homo sapiens

<400> 1

```
cgccacacat gtcaagctaa aggcagttgt tgggttacta acaggaccca gcgccttgca        60
aacatatgcg ctaagctgtg tatacagatg gcaggcagaa taatggagca ggcgcctttt       120
ataaagctct agctgctgcc tgtcttcaga cctgggaaat gaaactattc agacttgcg        179
```

<210> 2
<211> 397
<212> DNA
<213> Homo sapiens

<400> 2

cggcgggtaa tccccatctg atctgctgcc tgtgagctgc tgactgccgg ggcgcgcgag 60

cctagggggc acgggcagca gcgcggcgat gacctgatcc cgagcccgcc agggcgcccc 120

ttccttccct cccttccctc gctccctctg agcctgtgcg gagaccagct cggtgcggcg 180

gccccgggtg gaccatggcg gggagctcca gttaaaggcg tgtgggcgcc gagctggaga 240

ggacaccttc ggcatcgctg ccttttgagg gggcttattt aaactactga gtcattccag 300

atttaggatt ccctaaataa tcaccaactg tgcccgtctc gtgccggaaa gcaagcggaa 360

agagaaagag cgcccactct tgggaccccg cagatcg 397

<210> 3
<211> 549
<212> DNA
<213> Homo sapiens

<400> 3

cgcggagcca gtgcaacttc tccggcggga ccgcgcccaa tcttccccgc cgcagtcggg 60

gagccccggg ggccgccgag cacaggctgc ggaccgcggc gggcacgtgg gctcggctgc 120

agcgctgcgc caggcaccgg ctgctcggct ctgcccaacc tctccgctcc cgcctcggtc 180

cctgcctctg gggagagggt tcctcccccc ttccactttg caccagtccg agggaatttg 240

cggtcggtga cgcgcatcct taagagagcc acctgcagcg cgaggcgcgc cgctccaggc 300

ggcatcgcag ggctgggccg gcgcggcctg gggaccccgg gctccggagg ccatgccggc 360

gttggcgcgc gacggcggcc agctgccgct gctcggtaag gccccgctcg ctcgctcgca 420

gcccctcgcg gtccctcagc ccccaccccg cagtgtggac ccgggcgcgc gcctcctccg 480

gcccagccgc ctcgctcctc cccgcgctcg ctccttccat tgcctcccgc gcccctcccc 540

tctttcgcg 549

<210> 4
<211> 383
<212> DNA
<213> Homo sapiens

<400> 4

cgagtcggag tcaagcacag gcagtgggcg ggtggcctca gccggcacca tctggcacct 60

gcgattcatc gccactttcc attcgcccag aggtcagggg ggcagcaggc aaaggacacg 120

cagagctcgt gtgtcccagt tcctctgaaa ctttcagctg accgcaccct cccccgacaa 180

actctttaat catccatcaa gctagaagag tacagtgacc tctgcctggc ctttggagtt 240

tgcaaaagaa gcaagcaata aaaagcaaga ccaggttcgc ccctttgctt cctccacctc 300

ccaagagcag cgcgcagcgc ggcggtgcgg ccggaagggg ccgctgtttg ctcagcgcag 360

ccaccccgcc gctgcagccg ccg 383

<210> 5
<211> 409
<212> DNA
<213> Homo sapiens

<400> 5

cggcagcggc cgccccagtc ccagccccag ccccagcccc agccccgccc ccgccccgcg 60

ccccgccgga gacgcccgga tcggcggagc ctggcgcgag ccctcgcccc ctcgcctctc 120

ccgccgcgcc tccgcctgcc cgcccccgcc ggccgaggct gggctgcggg aggcggccgg 180

gcggccccga gcttcgctag ggcgaccaaa acaaaggcag catccggggc tgggtggatg 240

caaacaacca tgaaagactg ggttctcgct ctccccggct ctgctgctgc tgctgctgcc 300

gccgccgccg ctgctcctcc tcctgccgcc gccgctaggg ctccgctgtg aggggggaagc 360

aggggcgcag ctgctgggcg tgcatccgaa aggtgagagc cagagagcg 409

<210> 6
<211> 390
<212> DNA
<213> Homo sapiens

<400> 6

cgacccgccg gggctttccc gggcgctgct ctcctcctgc tgccccctcg ctaggacccg 60

gcggacgcct cgtctggttt tcacgccctc tagcccctac ccccacaccc ccaaaacaga 120

acagaccccc atccctgggc tggaggaccc gcctcttggc agccagctga gaaggcgccc 180

cggggagggg gaaactgaca tcccatctag agccgtccct cctcttcctc ccctcccgac 240

tctctgctcc tttcccgccc cagaagttca agggcccccg gcctcctgcg ctcctgccgc 300

cgggaccctc gacctcctca gagcagccgg ctgccgcccc gggaagatgg cgaggaggag 360

ccgccaccgc ctcctcctgc tgctgctgcg 390

<210> 7
<211> 244

<212> DNA
<213> Homo sapiens

<400> 7

cgaggctcac ctacccgggc ccgcgagtct cgcccagccc cagcctccca acccactctg 60

ccggccccaa cttaggcgag cgcagtcggg acccttctgt cggggctctt tgttcgccgc 120

ccctcggcgt tccagctctc aaccttgctc ccagcagccc ccgccggcgc actcgaccta 180

caggaaagtt gctcccgagc tgtggagggg cgtcatcgcc tcctagcaac gctcctagca 240

accg 244

<210> 8
<211> 395
<212> DNA
<213> Homo sapiens

<400> 8

cgccctcctc gccatggccc ggcctccaca tccgcccaca tctggccgca gcggggcgcc 60

cggggggagg ggctgaggcc gcgtctctcg ccgtcccctg ggcgcgggcc aggcggggag 120

gaggggggcg ctccggtcgt gtgcccagga ctgtccccca gcggccactc gggccccagc 180

cccccaggcc tggccttgac aggcgggcgg agcagccagt gcgagacagg gaggccggtg 240

cgggtgcggg aacctgatcc gcccgggagg cgggggcggg gcgggggcgc agcgcgcggg 300

gaggggccgg cgcccgcctt cctcccccat tcattcagct gagccagggg gcctaggggc 360

tcctccggcg gctagctctg cactgcagga gcgcg 395

<210> 9
<211> 1144
<212> DNA
<213> Homo sapiens

<400> 9

cgaacgcgcg ggaacattct ccgtgagaga aaaagcacgg gtggggggcg gttaccggtg 60
cttcccccgg tcgcctgggg gtcccgcacg cgccccgcgg gtgccgctgt ctggctgggg 120
tccctgggtg agccgcgggc accggcttcg cgtctccccg ggcgcgactc cgctttgcaa 180
gcgctcagca cacgggaaac cctcggaaaa cacacacgtg tgtaaagttt gttccacgca 240
gaaacaaagg acgcgtgggg gccgcttctg ggcctcggt cctttgttgg aaccccgcac 300
cgcagcccgg cccgcagcct cgccccgcaa ggggaagccg gccctgcaca ggcccggggc 360
ccggaaggcg ccgccgcaca gctctgcgcc cccgacccgc tccggccgcg cgctgggcca 420
ggcccttggc ctcaagcctc ccgggcggct ccgggccgga cccgggtctc cgtcgcggga 480
cgccagcctg tttgtgggtg ccgtgctcgg gcccgggcgg cccctgcccg acgcggcccc 540
acggagcccc tgcacgaacc ccgccgacct ggcccggggc ggcaagtcga ggtgcttagg 600
gcgccctggg cccggccaac cgtggaaaac ccggcccggg gcacgggggg gcgcgggggt 660
cgcagggcca gggcccaggc agggaacaga gatttttttc gcggggaggg gggtgggagg 720
tgtcaaagtt ttcggggaac ttttccactt gagattccac gaccctgcag tgccccctac 780
agaaagcggc agctggggag ggcgggcgcc cccgacgacc cccgactgcc agcggcggcg 840
cggccaggct gcctggagtt cttgttcctt tttttttttt ttttttcctt ttatttcctc 900
gtggttgttt gtcggataca tctttttcca gagtgggaaa aaaaaaaaaa aaagcgagcg 960
ccgccgcctg cagtagagcg tgctcggcct gcagaggcag tcgtctccaa cttttcctct 1020
cccgctccaa ctccccgcgc ccaaaacttc agttcttggg gagcccaaat ttgcctgccg 1080
cgcaacttcc actctaattc agcgaccaag ggccctggca gcggtttgca gcctgggggc 1140
cacg 1144

<210> 10
<211> 331
<212> DNA
<213> Artificial Sequence

<220>
<223> bisulfite-treated DNA

<400> 10

acgaataggg gtggagtata attttcgttc ggaagggtaa tttagtataa gttgagatag 60

tagtggcgag ggaagggtag tggggggtgg ggtgcggtgg gtgggggcgt ttgtttttta 120

taggattttt aggtttcgtc gtcgatttat aatttgttga aggagtaaag aatattttcg 180

gttttaagta gggtttttag tgtgtttatt gatgagtgaa agtcgttata tatgttaagt 240

taaaggtagt tgttgggtta ttaataggat ttagcgtttt gtaaatatat gcgttaagtt 300

gtgtatatag atggtaggta gaataatgga g 331

<210> 11
<211> 119
<212> DNA
<213> Artificial Sequence

<220>
<223> bisulfite-treated DNA

<400> 11

ttggagagga tattttcggt atcgttgttt tttgaggggg tttatttaaa ttattgagtt 60

atttagatt taggattttt taaataatta ttaattgtgt tcgtttcgtg tcggaaagt 119

<210> 12
<211> 159
<212> DNA
<213> Artificial Sequence

<220>
<223> bisulfite-treated DNA

<400> 12

gttcggttgt agcgttgcgt taggtatcgg ttgttcggtt ttgtttaatt ttttcgtttt 60

cgtttcggtt tttgtttttg gggagagggt tttttttttt ttttattttg tattagttcg 120

agggaatttg cggtcggtga cgcgtatttt taagagagt 159

<210> 13
<211> 319
<212> DNA
<213> Artificial Sequence

<220>
<223> bisulfite-treated DNA

<400> 13

agttcgggga tttatagggg tagtaaggcg gtagagtcgc ggagttagtg taattttttc 60

ggcgggatcg cgtttaattt ttttcgtcgt agtcggggag tttcgggggt cgtcgagtat 120

aggttgcgga tcgcggcggg tacgtgggtt cggttgtagc gttgcgttag gtatcggttg 180

ttcggttttg tttaattttt tcgttttcgt ttcggttttt gtttttgggg agagggtttt 240

tttttttttt tattttgtat tagttcgagg gaatttgcgg tcggtgacgc gtatttttaa 300

gagagttatt tgtagcgcg 319

<210> 14
<211> 247
<212> DNA
<213> Artificial Sequence

<220>
<223> bisulfite-treated DNA

<400> 14

gtggttttag tcggtattat ttggtatttg cgatttatcg ttattttttta ttcgtttaga 60

ggttaggggg gtagtaggta aaggatacgt agagttcgtg tgttttagtt tttttgaaat 120

ttttagttga tcgtattttt tttcgataaa ttttttaatt atttattaag ttagaagagt 180

atagtgattt ttgtttggtt tttggagttt gtaaaagaag taagtaataa aaagtaagat 240

taggttc 247

<210> 15
<211> 299
<212> DNA
<213> Artificial Sequence

<220>
<223> bisulfite-treated DNA

<400> 15

aattggaggt agtagagttt gggagtagtt tttacggcgg tagcggtcgt tttagtttta 60

gttttagttt tagttttagt ttcgttttcg tttcgcgttt cgtcggagac gttcggatcg 120

gcggagtttg gcgcgagttt tcgtttttttc gttttttttcg tcgcgttttc gtttgttcgt 180

tttcgtcggt cgaggttggg ttgcgggagg cggtcgggcg gtttcgagtt tcgttagggc 240

gattaaaata aaggtagtat tcggggttgg gtggatgtaa ataattatga aagattggg 299

<210> 16
<211> 290
<212> DNA
<213> Artificial Sequence

<220>
<223> bisulfite-treated DNA

<400> 16

gattcgtttt ttggtagtta gttgagaagg cgtttcgggg agggggaaat tgatatttta 60

tttagagtcg tttttttttt tttttttttt tcgatttttt gttttttttt cgttttagaa 120

gtttaagggt tttcggtttt ttgcgttttt gtcgtcggga ttttcgattt ttttagagta 180

gtcggttgtc gtttcgggaa gatggcgagg aggagtcgtt atcgtttttt tttgttgttg 240

ttgcgttatt tggtggtcgt tttgggttgt aagttgttcg ggttttttta 290

<210> 17
<211> 257
<212> DNA
<213> Artificial Sequence

<220>
<223> bisulfite-treated DNA

<400> 17

ggggatatcg agttaaggtt tgtagcgagt gatttgttcg gggaggcgcg ggatcgaggt 60

ttatttattc gggttcgcga gtttcgttta gttttagttt tttaatttat tttgtcggtt 120

ttaatttagg cgagcgtagt cgggattttt ttgtcggggt tttttgttcg tcgtttttcg 180

gcgttttagt ttttaatttt gtttttagta gttttcgtcg gcgtattcga tttataggaa 240

agttgttttc gagttgt 257

<210> 18
<211> 410
<212> DNA
<213> Artificial Sequence

<220>
<223> bisulfite-treated DNA

<400> 18

agagttagtc gtcggaggag tttttaggtt ttttggttta gttgaatgaa tgggggagga 60

aggcgggcgt cggtttttttt tcgcgcgttg cgttttcgtt tcgttttcgt ttttcgggcg 120

gattaggttt tcgtattcgt atcggttttt ttgtttcgta ttggttgttt cgttcgtttg 180

ttaaggttag gtttgggggg ttggggttcg agtggtcgtt gggggatagt tttgggtata 240

cgatcggagc gttttttttt ttttcgtttg gttcgcgttt aggggacggc gagagacgcg 300

gttttagttt tttttttcgg gcgtttcgtt gcggttagat gtgggcggat gtggaggtcg 360

ggttatggcg aggagggcgg gcgtttagag gggaagagat tttttttttt 410

<210> 19
<211> 237
<212> DNA
<213> Artificial Sequence

<220>
<223> bisulfite-treated DNA

<400> 19

ggaaaatata tacgtgtgta aagtttgttt tacgtagaaa taaaggacgc gtgggggtcg 60

tttttggggt ttcggttttt tgttggaatt tcgtatcgta gttcggttcg tagtttcgtt 120

tcgtaagggg aagtcggttt tgtataggtt cggggttcgg aaggcgtcgt cgtatagttt 180

tgcgttttcg attcgtttcg gtcgcgcgtt gggttaggtt tttggtttta agtttttt 237

<210> 20
<211> 32
<212> DNA
<213> Artificial Sequence

<220>
<223> primer

<400> 20
agggtaattt agtataagtt gagatagtag tg 32

<210> 21
<211> 31
<212> DNA
<213> Artificial Sequence

<220>
<223> primer

<400> 21
ctccattatt ctacctacca tctatataca c 31

<210> 22
<211> 23
<212> DNA
<213> Artificial Sequence

<220>
<223> primer

<400> 22
ttggagagga tattttcggt atc 23

<210> 23
<211> 18
<212> DNA
<213> Artificial Sequence

<220>
<223> primer

<400> 23
actttccgac acgaaacg 18

<210> 24
<211> 18
<212> DNA
<213> Artificial Sequence

<220>
<223> primer

<400> 24
gttcggttgt agcgttgc 18

<210> 25
<211> 25
<212> DNA
<213> Artificial Sequence

<220>
<223> primer

<400> 25
actctcttaa aaatacgcgt caccg 25

<210> 26
<211> 29
<212> DNA
<213> Artificial Sequence

<220>
<223> primer

<221> misc_feature
<222> (16)..(16)
<223> n stand for c or t

<400> 26
gtggttttag taggtnttat ttggtattt 29

<210> 27
<211> 31
<212> DNA
<213> Artificial Sequence

<220>
<223> primer

<221> misc_feature
<222> (1)..(1)
<223> n stand for a or g

<400> 27
naacctaatc ttacttttta ttacttactt c 31

<210> 28
<211> 27
<212> DNA
<213> Artificial Sequence

<220>
<223> primer

<400> 28
aattggaggt agtagagttt gggagta 27

<210> 29
<211> 27
<212> DNA
<213> Artificial Sequence

<220>
<223> primer

<400> 29
cccaatcttt cataattatt tacatcc 27

<210> 30
<211> 28
<212> DNA
<213> Artificial Sequence

<220>
<223> primer

<400> 30
gattcgtttt ttggtagtta gttgagaa 28

<210> 31
<211> 25
<212> DNA
<213> Artificial Sequence

<220>
<223> primer

<400> 31
taaaaaaacc cgaacaactt acaac 25

<210> 32
<211> 25
<212> DNA
<213> Artificial Sequence

<220>
<223> primer

<221> misc_feature
<222> (9)..(9)
<223> n stand for a or g

<400> 32
ggggatatng agttaaggtt tgtag 25

<210> 33
<211> 26
<212> DNA
<213> Artificial Sequence

<220>
<223> primer

<221> misc_feature
<222> (8)..(8)
<223> n stand for c or t

<400> 33
acaactcnaa aacaactttc ctataa 26

<210> 34
<211> 26
<212> DNA
<213> Artificial Sequence

<220>
<223> primer

<400> 34
ttagagtggt agttggggga tagttt 26

<210> 35
<211> 26
<212> DNA
<213> Artificial Sequence

<220>
<223> primer

<400> 35
aaaaaaaaat ctcttcccct ctaaac 26

<210> 36
<211> 27
<212> DNA
<213> Artificial Sequence

<220>
<223> primer

<400> 36
ggaaaatata tacgtgtgta aagtttg 27

<210> 37
<211> 25
<212> DNA
<213> Artificial Sequence

<220>
<223> primer

<400> 37
aaaaacttaa aaccaaaaac ctaac 25

<210> 38
<211> 25
<212> DNA
<213> Artificial Sequence

<220>
<223> primer

<400> 38
agttcgggga tttatagggg tagta 25

<210> 39
<211> 28
<212> DNA
<213> Artificial Sequence

<220>
<223> primer

<400> 39
cgcgctacaa ataactctct taaaaata 28

<210> 40
<211> 26
<212> DNA
<213> Artificial Sequence

<220>
<223> primer

<400> 40
gagttggaga ggatattttt ggtatt 26

<210> 41
<211> 21
<212> DNA
<213> Artificial Sequence

<220>
<223> primer

<400> 41
ctttccaaca caaaacaaac a 21

<210> 42
<211> 23
<212> DNA

<213> Artificial Sequence

<220>
<223> primer

<400> 42
tgtgggtttg gttgtagtgt tgt 23

<210> 43
<211> 29
<212> DNA
<213> Artificial Sequence

<220>
<223> primer

<400> 43
actctcttaa aaatacacat caccaacca 29

<210> 44
<211> 20
<212> DNA
<213> Artificial Sequence

<220>
<223> primer

<400> 44
attattctac ctaccatcta 20

<210> 45
<211> 19
<212> DNA
<213> Artificial Sequence

<220>
<223> primer

<400> 45
ggggtagtag gtaaaggat 19

<210> 46
<211> 17
<212> DNA
<213> Artificial Sequence

<220>
<223> primer

<221> misc_feature
<222> (5)..(5)
<223> n stand for a or g

<400> 46
ccccnaatac taccttt 17

<210> 47
<211> 20

<212> DNA
<213> Artificial Sequence

<220>
<223> primer

<400> 47
ttttggtagt tagttgagaa 20

<210> 48
<211> 19
<212> DNA
<213> Artificial Sequence

<220>
<223> primer

<400> 48
tccaaaacaa ctttcctat 19

<210> 49
<211> 17
<212> DNA
<213> Artificial Sequence

<220>
<223> primer

<400> 49
ggggatagtt ttgggta 17

<210> 50
<211> 15
<212> DNA
<213> Artificial Sequence

<220>
<223> primer

<400> 50
aaggaagagt ggggg 15

<210> 51
<211> 18
<212> DNA
<213> Artificial Sequence

<220>
<223> primer

<400> 51
ccaacaaccc atacctaa 18

<210> 52
<211> 331
<212> DNA
<213> Homo sapiens

<400> 52

acgaataggg gtggagtata attttcgttc ggaagggtaa tttagtataa gttgagatag 60
tagtggcgag ggaagggtag tggggggtgg ggtgcggtgg gtgggggcgt ttgtttttta 120
taggattttt aggtttcgtc gtcgatttat aatttgttga aggagtaaag aatattttcg 180
gttttaagta gggtttttag tgtgtttatt gatgagtgaa agtcgttata tatgttaagt 240
taaaggtagt tgttgggtta ttaataggat ttagcgtttt gtaaatatat gcgttaagtt 300
gtgtatatag atggtaggta gaataatgga g 331

<210> 53
<211> 119
<212> DNA
<213> Homo sapiens

<400> 53

ctggagagga caccttcggc atcgctgcct tttgaggggg cttatttaaa ctactgagtc 60
attccagatt taggattccc taaataatca ccaactgtgc ccgtctcgtg ccggaaagc 119

<210> 54
<211> 159
<212> **DNA**
<213> Homo sapiens

<400> 54

gctcggctgc agcgctgcgc caggcaccgg ctgctcggct ctgcccaacc tctccgctcc 60
cgcctcggtc cctgcctctg ggagagggt tcctcccccc ttccactttg caccagtccg 120
agggaatttg cggtcggtga cgcgcatcct taagagagc 159

<210> 55
<211> 319
<212> DNA
<213> Homo sapiens

<400> 55

agttcgggga ctcacagggg cagcaaggcg gcagagccgc ggagccagtg caacttctcc 60
ggcgggaccg cgcccaatct tccccgccgc agtcggggag ccccgggggc cgccgagcac 120
aggctgcgga ccgcggcggg cacgtgggct cggctgcagc gctgcgccag gcaccggctg 180
ctcggctctg cccaacctct ccgctcccgc ctcggtccct gcctctgggg agagggttcc 240
tcccccttc cactttgcac cagtccgagg gaatttgcgg tcggtgacgc gcatccttaa 300
gagagccacc tgcagcgcg 319

<210> 56
<211> 246
<212> DNA
<213> Homo sapiens

<400> 56

gtggcctcag ccggcaccat ctggcacctg cgattcatcg ccactttcca ttcgcccaga 60

ggtcaggggg gcagcaggca aaggacacgc agagctcgtg tgtcccagtt cctctgaaac 120

tttcagctga ccgcaccctc ccccgacaaa ctctttaatc atccatcaag ctagaagagt 180

acagtgacct ctgcctggcc tttggagttt gcaaaagaag caagcaataa aaagcaagac 240

caggtt 246

<210> 57
<211> 299
<212> DNA
<213> Homo sapiens

<400> 57

aattggaggc agcagagcct gggagcagcc tccacggcgg cagcggccgc cccagtccca 60

gccccagccc cagccccagc cccgccccc g ccccgcgccc cgccggagac gcccggatcg 120

gcggagcctg gcgcgagccc tcgccccctc gcctctcccg ccgcgcctcc gcctgcccgc 180

ccccgccggc cgaggctggg ctgcgggagg cggccgggcg gccccgagct tcgctagggc 240

gaccaaaaca aaggcagcat ccggggctgg gtggatgcaa acaaccatga aagactggg 299

<210> 58
<211> 290
<212> DNA
<213> Homo sapiens

<400> 58

gacccgcctc ttggcagcca gctgagaagg cgccccgggg agggggaaac tgacatccca 60

tctagagccg tccctcctct tcctcccctc ccgactctct gctcctttcc cgccccagaa 120

gttcaagggc ccccggcctc ctgcgctcct gccgccggga ccctcgacct cctcagagca 180

gccggctgcc gccccgggaa gatggcgagg aggagccgcc accgcctcct cctgctgctg 240

ctgcgctacc tggtggtcgc cctgggctgt aagttgctcg ggttcctcta 290

<210> 59
<211> 257
<212> DNA
<213> Homo sapiens

<400> 59

ggggacaccg agccaaggcc tgcagcgagt gacctgcccg gggaggcgcg ggaccgaggc 60

tcacctaccc gggcccgcga gtctcgccca gccccagcct cccaacccac tctgccggcc 120

ccaacttagg cgagcgcagt cgggaccctt ctgtcggggc tctttgttcg ccgcccctcg 180

gcgttccagc tctcaacctt gctcccagca gcccccgccg gcgcactcga cctacaggaa 240

agttgctccc gagctgt 257

<210> 60
<211> 388
<212> DNA
<213> Homo sapiens

<400> 60

cctaggcccc ctggctcagc tgaatgaatg ggggaggaag gcgggcgccg gcccctcccc 60

gcgcgctgcg cccccgcccc gcccccgcct cccgggcgga tcaggttccc gcacccgcac 120

cggcctccct gtctcgcact ggctgctccg cccgcctgtc aaggccaggc ctgggggct 180

ggggcccgag tggccgctgg gggacagtcc tgggcacacg accggagcgc ccccctcctc 240

cccgcctggc ccgcgcccag gggacggcga gagacgcggc ctcagcccct ccccccgggc 300

gccccgctgc ggccagatgt gggcggatgt ggaggccggg ccatggcgag gagggcgggc 360

gcccagaggg gaagagattc ctcccctt 388

<210> 61
<211> 237
<212> DNA
<213> Homo sapiens

<400> 61

ggaaaacaca cacgtgtgta aagtttgttc cacgcagaaa caaaggacgc gtgggggccg 60

cttctggggc ctcggtcctt tgttggaacc ccgcaccgca gcccggcccg cagcctcgcc 120

ccgcaagggg aagccggccc tgcacaggcc cggggcccgg aaggcgccgc cgcacagctc 180

tgcgcccccg acccgctccg gccgcgcgct gggccaggcc cttggcctca agcctcc 237